# EUROPEAN PATENT APPLICATION

(11) **EP 2 055 775 A1**
(43) Date of publication of application: **06.05.2009**
(21) Application number: 07832871.3
(22) Date of filing: 30.11.2007
(51) Int. Cl.: C12N 15/09, C12Q 1/68, G01N 21/78

(54) **PRIMER SET FOR AMPLIFICATION OF CYP2C9 GENE, REAGENT FOR AMPLIFICATION OF CYP2C9 GENE COMPRISING THE SAME, AND USE OF THE SAME**

(30) Priority: 30.11.2006 JP 2006322959
(71) Applicant: Arkray, Inc., Kyoto-shi, Kyoto 601-8045 (JP)
(72) Inventor: HIRAI, Mitsuharu c/o Arkray, Inc.,, Kyoto-shi, Kyoto 601-8045 (JP); MAJIMA, Satoshi c/o Arkray, Inc.,, Kyoto-shi, Kyoto 601-8045 (JP)
(74) Representative: Behnisch, Werner
(86) International application number: PCT/JP2007/073206
(87) International publication number: WO 2008/066163

(57) **Abstract**

A primer set for amplifying a region including a site to be detected of CYP2C9*3 in the CYP2C9 gene by a gene amplification method is provided, wherein the primer set can amplify the region specifically A pair of primer set is used including a forward primer consisting of the base sequence of SEQ ID NO: 4 as well as a reverse primer consisting of the base sequence of SEQ ID NO: 17. The use of this primer set makes it possible to specifically and efficiently amplify a target region including the site where a polymorphism, CYP2C9*3, of the CYP2C9 gene is generated.

[Sequence Table] TF07040-01.ST25.txt

SEQUENCE LISTING

<110>  ARKRAY, Inc.

<120>  Primer set for amplifying CYP2C9 gene, reagent for amplifying CYP2C9 gene
containing the same, and the uses thereof

<130>  P24713

<150>  JP2006-322959
<151>  2006-11-30

<160>  30

<170>  PatentIn version 3.1

<210>  1
<211>  60723
<212>  DNA
<213>  Homo sapiens

<400>  1
ttgtggagga agtgagtgcc agatctaatc agtaatgttg catcccttca tttgttttta      60

aagtttgttt gctgtaggct gtctctgtgc cacttattag cccaaggtat taacgtaagg     120

tctattcaag accttagtga agactttgct gtggcatgtg tgttaagtgt ctaatttttc     180

ccacatagcc agtttttaaa tgtcttagtc tttaatgtct atctcccaaa agggaaaaaa     240

gagaaacaca aataggagag ggaaaaggtg tcagtccttt aaataccctg gaagtcactt     300

gttgggggag cttgccaaga cttgggggaa ttgtagcaat aatgggttcc caatctgtgt     360

ctgtacctaa gttttcaaaa gcagcagtca tcagaggaca ggtcttcaat attcagaaga     420

taggttcttt tttgcccatc ccagctcctg caagctgtga ataagctgct ccaggaacat     480

gtgtacagct tcctgccagg ggctgaagta tggggcattg gtaggtgcca ctgtgctaag     540

aactgaaatt gacaaaattg gttttttttt ttatagattt ttttaaaatt aatttttatt     600

tttttgagac agagtctcac tctgtcagcc aggctggagt gcagtggtgt gatcttggct     660

cactgcaacc tctgcctcct gggttcaagt gattcttggg cctcagtctc ccaagtagct     720

gggactacag atgtgcacca ccacactggc taattttttg tattttcaga agagacaggg     780

tttcttctta aatcatttta ttaaaaacta ggacacaaaa cacattagcc taggagtatg     840

caaggtcagg atcaccaata tcaccatatt ctactgccac atcttgtccc actgtaaggt     900

tttcaggggc aataacacat atgaagctgt aatcgcctat gtaacattgc cttcttctgg     960

aataactcct gaagtacctg cctgaggttg tttaatagtt aacttaaaag aaaatgagtc    1020

gtagaagaac acaacacttt gaaataacag taaaagtata gtatagtaaa tacataaacc    1080

agtaacagag tcatttacta tagttattga gtattatgta ctgtacctga tatgtgctat    1140

atttttatac aactggcagt gcagtaggtt tgtttacatc agtgtcacca catacacatg    1200

aaaaatgtgt tgtaccaaga tattatgatg agtatgagta tctaggtggc agaaatcatc    1260

tttcagctcc attgtaatct tttttctgag acagagttgc actctgtcac acaggctgaa    1320

gtgcagtggt gtgatcttgg ctcactgcaa cctccatctc tgagtttcaa gctattctcc    1380

tgcctcagcc tccctagtag ctgggactac aggtgcctgc caccacagcc agctaatttt    1440

tgtactttta gtaaaaatga aatttcacca tgttggccag gctgttctcg atctcctgac 1500
ctcaagtgat ccacccgcct tggcctccca aagtgctggg attataggtg tgagccacca 1560
tgcccagcct cagccccatt ataatcttat ggaacaacat agtaaatgaa atcacctgtt 1620
gaacaaaatg ttggtatatg gtgcatgact gtattaaaag tattggaaaa aatcccaccc 1680
atcttgattt gtgtatctgg caaaattctc cttcacaagt gatggagaaa taaagattct 1740
cacacaaaaa aattgagaaa atttatctct agttcctgcc ttgcaaaaaa tgttaaataa 1800
agttcttcag agagaagaaa atggtgtaag tcaatcactg atctacatga ggaaaggaag 1860
ggagttagtg aaggaataat aaaaggaaca taacattttt attttcttat tgattggtct 1920
aacagacaac acttttttca aaataataac aataacatgg ccctgcaaaa gtggctcaca 1980
cctgtaatcc cagcactttg ggagcccgag gagggcggat cacctgaggt caggagttcc 2040
agaccaacct gaccaacata gagaaacact gtctctacta aaaatacaaa attagcaagg 2100
agtggtggca cattcctgta atcccagcta cttgggaggc tgaggcagaa gaattgcttg 2160
aaggcaggag gcagaagttg cagtgagccg agatcatact attgcacttc agcctgtgta 2220
acaagagtga aactccatct caaaaacaaa acaaaacaaa acaaacaaaa cagcaacaac 2280
aaaaatatat atttggtgat tacagtttat ggataaatga aataaatgac agtgatgata 2340
tatgaagaag ggaggaaaga agtaagaata tttttttaca ggtaagtgca ctacctatga 2400
agtagtatag tgctatttta atgtagactt gggttaattg taaacacata ttgcacataa 2460
tacggaaact acaaaaaagt aagaaaaaac aagtaatatt aaggagaaaa agaaaataga 2520
ctcatataac atgctcaatt aaaaccacag aaggcagaaa aagagttgag gacaaaaata 2580
gaaacaacga acaagggcaa tgagtagaaa acagtaacgc atatatggta gataatagtt 2640
caactatatt aaagtcattt taaccgttgg aaagacagag acggtcagag tagataaaaa 2700
aacaagagcc aaatatgtgt atgttgtcta taagagaccc atttgagata taaatataca 2760
tatagatttc aagtaaatga aggagaaaaa tataccatgc taacaccaac caaaaggaaa 2820
tgggaatagc tatattagtt tcagatgaag ccatctccag agtaagaaaa aattattaga 2880
ggttaggaag agtgttatat aatgataaag agttaaattc tctaaggaga aataataaag 2940
tgtatgcacc caacaataag gtcaaaatac atgaggcaaa aactgataga actacaatga 3000
gaaatagatg actccactat tacagctgga gaattaaaca ctcctctatt aaaaatggac 3060
agattcagca tgcagaaaat cattaaagac agatttgaac tgaatagtac catcaatcaa 3120
ctgatttgaa ttggcattta tagaatactt taccctacaa aagcagaata cacattcttc 3180
tcaagctcac atagaacatt caccaatata gaccacattc tgggcaacaa aacatatctt 3240
aggaagttta aaagaatgtg aatcatacaa tgtctgctat gagaccacag ttgaattaaa 3300
ctagaaattg ataacagata ttgagctgga atatcacaaa atacttagag agtaaacagc 3360
acatttctta ataaaatatg ggtaaaataa taaatctcaa gagggattaa aatatttgga 3420
acttaacaaa ataaaaatag aactttaaaa aatttgtagg atgtagtgaa agcagtggtt 3480

agagggaaac ttctagcatt gggggcataa attagaaaag aaaaggatac aaagtcagta 3540
atctaaacat ccaccttagg gaaccagaga aagaaggaca aattatatcc aaagaaagca 3600
gagcaaaaga aacaataaaa gagcagaaat caatgaaatt gaaaacaaga aatcaataga 3660
gaaaattagt aaaaacaaaa gctggttatt tgaaaattat atcaatatat atctagccag 3720
cctaaccaag ataaaaaggg agaatacaga ttttcctatc atcaaaatta aaagaagaac 3780
catcactatt gataccatgg acattaaaag gataataaat gaatattata aagaactcca 3840
tgcccacaga ttatataact tggataaaat ggatcaattc cataaagaaa caatcttcta 3900
agtctcacac aagaagaaat agataatttg aataggcctt tatttattaa agtaataaaa 3960
taaaaaataa ataacctcca aaagagaaaa caccagaccc atataggtcc actggtaaat 4020
tctaccaaac atttatagaa gaattatacc cgttctctac aatctcttct agaagtagta 4080
ccagaggaaa ttcttctatt cttaactcat tttatggggc cagcattact ctaatactca 4140
aatcagacaa ggacattgta agaaagcaaa actatatatc agtatatcta atgaaaatag 4200
gtgcaaacct caataaaaga ttagcaaatt gcatccaatc gtgtagaaaa actgtataca 4260
ccataactaa gtgggattta ttttagaaat gcaagagtta ctcaacatat gaaaatcaaa 4320
tattttactt catcacatta gttactaaag aagaaaagtc tcatcatcat atccacaggt 4380
gcagaaaaaa aatttgccta aatccagcac acattcatga taaaaactct cagcgaacta 4440
agaatagagg aggaactttc tcaatttaat aaagaatatg tgcaaaaaac ctaccgctaa 4500
catcattatt ggtgagaaac ttgaaatttt ctggctaata acaagaataa agtgagatgt 4560
tccatgaaac tactcctatt caacatcgta ctagaaaccc tttttaatga gataacaaga 4620
aaaagaaata aaagtgatac caatcaggaa taaagaaata aaactattgt gtttagatcg 4680
catgattgtt tatgtagaaa atctcaaaga atcaaaaaac ctccaaaaat aaaaaaaaca 4740
aaacaaaaca aaaagaaaac aacaataatg aaaaaactcc taaaagtaat aagctattgt 4800
agtaaggttg caggatacaa ggaaatcaaa ttcttttctc tatattaaca ataaataatt 4860
agaatttgga attaaaaaca tagtatcatt tatattagca ccaaaaatat gaaatcccta 4920
ggtggaagtc tgtcaaaata catatatcac ttgaagaaaa ctacaaaact ctgatgaaag 4980
aaaacaaaga tataaataaa tacacaaata tttcatgttc atggatatga agaccgttgt 5040
tagtcttttt gatttgatct atagatcaac acaataagaa tcaaactcca ggaaagtttt 5100
attattttta ttatttttta tctttagatg ctaacaatct gattgtaaag tttatgtgga 5160
gaggccaaaa aacccagaat agccaacgtt aaggagaaca aagtcaaatg actgacctta 5220
cccaattcaa cttcaagact tactatgaag ctaatcaaga cagtgtgcaa ttggtgaaaa 5280
aagtcaaata gattaatgca agtgagtaaa gagtctgaag tagatcatca gaaatatagt 5340
caactgatct ttgaaaaaaa aggaaagaca attcaatggg ggaaaaatgg tcttttcaac 5400
gaagactaat ggagtaactg gaggttcaca tacggaaaaa tgaatctgga aatagacctt 5460
acacctttaa tgaaagttaa ctaaaaatat attatagatc taagccgggt gcagtggctt 5520
atgccagtat tcccagtact ttgggaggct gaggtaggca gatcacaggt caggagatcg 5580

agaccatcct ggctaacacg gtgaaacccc catctctact aaaaatacaa aaaattagct 5640
gggcatggtg gcacgcacct gtagtcccag gttgtcaaga ggctgaggca ggagaatcgc 5700
ttgaactcag gaggcagagg ttgaagtgag ccgagttgag agccactgca ctccagccta 5760
ggtgacagag tgagactcag tctcaggaaa aaaaaaaaaa aaaaaaaaaa tatatatata 5820
tatatatgtg tatatatata tatatgtgta tatatatata tatgtgtata tatatatata 5880
tatatatata cgtgtgtgta tatatatata tacatatata tattagatcc aaatataaaa 5940
tgcaaaacta tgaatctctg atagtataag ataggagaaa ataaagatga ccttgatcag 6000
caatggtttt taaaatacta caatgaaggt ataatccatg aaataaagaa tgactaagct 6060
ggaccttatt aaaatttaaa cttctactct gcaaaagaca ctatcaaggg aatgagatga 6120
aaagacagag actgggagaa atactttcaa aagatatatc tgataaacga cttctctcca 6180
aaaattttta aaactccaca attagaaatc agacaccttg atttaaaaaa tgggcaacag 6240
atctgaacag acacctcacc caagaagaaa tacagatggc aaataaatat atgaaaaatg 6300
ctcatcatta aacgtcacta cggacctgca aactaaaacc ccaatgagat acaactacac 6360
acttatacaa tggctaaaat ccaaaacact gataaaacca aatgttgaca aggatgtgca 6420
gcaatgagaa ctaagtcatt gctggtggaa atgcaaaatg ataaagacac ttcaggagac 6480
agatgggcag tgcctaaaaa aactaaaata tgtttaccac attatctggt agttgtgctc 6540
tttggtattt gccaaataag ttgaaaatac atttccacac aaaaatctgc acatggatat 6600
ttatagcagc tttattcatc attgtcaaaa attagaaatc accaagatgt tcttcaatag 6660
ataaatgaat agacaacgtg ataaacctat acaatgaatg ttatttagtg ttaacaagga 6720
atgaactatc aagccatgaa aagacatgca gaaactttat acatatgagt aagttaaatc 6780
ttaaaaggct acatactgta tgattccaac cgtattacat tttggaaaag gcaaaactat 6840
gcctacactg caaagattat ttgttgctag ggcttgcaag gaagggagag agaacacgta 6900
aagcacagag gttcttaggg cagtaaagct attttatgtg atattacaat ggcacatgtc 6960
attatgcatt agtcaaatcc catagaatgt acaacacaaa gaatgaaccc tacataaact 7020
atgagctttg gttgatgatg atgcgtcaat gttggttcat tgattgtaac taatgagagt 7080
tcttgattgt gagagagact gtgtgtgggt gcagggaaag aggcatgtgg taattatata 7140
ctttccactt agtgttgcca gaaaccccaa actgtttcaa aagcctactc taatccacca 7200
ttctagtcaa gctgaggttt gtattactca gtgactgtgg agggcttaat gttgatactc 7260
ccctgatcat ttggactgag atgtgaattc tatgagatgg gatgtgacat gtcactgaat 7320
tgggagttga aaaactggga ttctaagaaa agtcttggct gggcgttgtg gctcatgcct 7380
gtaatcccag cactttggga gacctaggtg ggtggatcac aaggtcggga gatcaagatc 7440
atcctgccca acatggtgaa accctgtctg tactaaaaat acaaaaaaat tagctgggcg 7500
tggtggtggg cacctgtagt cccagctact tgggaggctg aggcaggaga atggcatgaa 7560
cccaggagct ggaaattgca gtgagccgag atcgcaccac tgcactccag cctgggtgac 7620

agagcgagac tctgtctaaa aaaaaaaaaa gaaaagaaaa agtcttatac tgaggcattg 7680
tgattgtgat actttgtctc actgagtcaa taattgctca tttcttaaaa aaaaaaaagc 7740
aaagtttaga gtagttgatc tcagatatcc cttctatcta cacattatct ataattcttt 7800
ctttctgtaa actgaaaggt ctaggaagga gccgcagctc agcaggagag aggaggagct 7860
gagctgggac ccctacctcc tgaggaatga aatgattatt ataaagacag caaccgagct 7920
tattttaccc aaaataaggt agtatatttc tgttagagtt tagagtttca tgagtcaggg 7980
accaagttat tgcttttctt tgccctgtat aaaggcttct ccaaggcctt tgacttacct 8040
aagtactaaa tgttataaaa ccaaactctt ctgacctctc aatctagtca actggggctg 8100
taattattaa tgaaattaat gtttattttg aaaataattt actagactga attacgaaat 8160
cctgaatcat tgtacactat cagtaaatat tggtggaccc aactgaactg aatgttttgc 8220
ttgaaatgaa acctttgaga tgcagggctt atgggttcta gtcccagctc tagcactagc 8280
agacagcatg ttcttggcta agatactgaa tcttcaaggc tcagcttcct cattccggaa 8340
atgggtcaat tttattgtaa gcagaggtaa ttgagagatt caaaagggac atgaggtgta 8400
acaattctct gtaaattgtt agaatccctg ttaaaaatga ccagtaaagc tttgtgcaac 8460
tgtgtcttga cataacttta tttttcttaa taaaagaaat ggaaataacc tcactaggga 8520
atttagaaca aatatgatga tatctttaaa gaaaatggct ttgcacaagt attgacatta 8580
atgatctagt aaagtgtatc tttctagttg tatttagatc ctcaactcag tatgtcagct 8640
cctgttaagg tctatacatt gtggtggttc tgtgctgtgg gtccatttag tgatttccct 8700
acctcccatc ttttattgca tccacaactg tggttctgtc cataatttcc tttgctttct 8760
gtgcattatt acatcatatc tgaaaatgag aaaccaaaaa caatagaaag cagccatgtc 8820
tggaggtgac tggggggtcg agaagcccta gtttctcaaa cccttagcac caaatttttc 8880
cctcagttac actgagcgtt tcacttctgc agtgatggag aagggagatc ccttatttct 8940
tctcatgagc atctctggtg ctgtttccct tagagacaaa taaggggttc tatttaatgt 9000
gaagcctgtt ttatgaacag aataaatgtg gtgtatattc agaataacta atgtttggaa 9060
gtttgtttta ttttgctaaa attgttctca aggcagctct ggtgtaagag ataatacacc 9120
acgatgggca tcagaagacc tcagctcaaa tcccagttct gccagctatg agctgtgtgg 9180
caccaacagg tgtcctgttc tcccagggtc tcccttttcc catttgaaaa ataaaaaata 9240
acaattcctg ccttcaggaa ttttttttag ggggtttaat ggtaaaggtg tttatatctg 9300
ctaaggtaat ttacttgata tatgtttggt tatttaagat atatgagtta tgttagctat 9360
ttcatgttta ggctgctgta tttttagtag gctatattaa atatttgaaa ggatttcatt 9420
ataaagaaca aagtctccta atctttgata tagcattgac atacttttta aatatacaag 9480
gcatagaata tggccatttc tgttaaatca tatattccca actggttatt aatctaagaa 9540
ttcagaattt tgagtaattg cttttgcatc agattattta cttcagtgct ctcaattatg 9600
atggtgcatt agaaccatct gggttaacat ttgtttttta ttaccaatac ctaggctcca 9660
accaagtaca gtgaaactgg aatgtacaga gtggacaatg gaacgaagga gaacaagacc 9720

aaaggacatt ttatttttat ctgtatcagt gggtcaaagt cctttcagaa ggagcatata 9780
gtggacctag gtgattggtc aatttatcca tcaaagaggc acacaccgaa ttagcatgga 9840
gtgttataaa aggcttggag tgcaagctca tggttgtctt aacaagaaga gaaggcttca 9900
atggattctc ttgtggtcct tgtgctctgt ctctcatgtt tgcttctcct ttcactctgg 9960
agacagagct ctgggagagg aaaactccct cctggcccca ctcctctccc agtgattgga 10020
aatatcctac agataggtat taaggacatc agcaaatcct taaccaatgt aagtatgctc 10080
cttcagtggc ttgcaaaagg taagtaaatt cacctgtatt ttttaaataa agtgtatccc 10140
tagaggtaca tgttacaaga ggtaatggta aagtaaaata ctttgaaagg cttttgttgc 10200
cttttccagt ctgtcagtgt cagaaatagt ggaatgaaac catgtatttt gtgagtagag 10260
aaagatttgg gtctttgcat gttagattca aaataacaag tgtcaatagt ttgaaaagct 10320
gtgttccttc ttcatttcat aaccatttgc tataattttt ggctgaaggt aaatggtaag 10380
gtattgtggg atctggtcag cagcccacaa agcaactggg ctctctcttt tttcccaggt 10440
ggatcggcag gttgagaaat aatagacaca cacaagatag tgaaagctgg gtccaggggg 10500
gtcaccgcct tctggtccca tggtgccaag aatgcactgg atataccagc atttattatt 10560
aagtttagtg agggcagggg taggttagtg agggatttag ggtcatttga ttatgaggtg 10620
agatggtcac atggggatga agtaattctt taacataaca tttgtatgta gaagtacagt 10680
acatttgtat gtagaagtac agtatacgga gataagaatt tacaatatag tgtgtgcgtc 10740
agtaatttct aacagagcct taaaacagaa acacaatctt tccataacct atgattagca 10800
agatattaat cagcagtaac aattgcaaca aaagctggtt acaaacaatc catggaaata 10860
ggatgtgaag ctagacaacc agttagacca gaaattctca gaagggagta tgccttaacc 10920
ctaaagaggc ctagaagagc catggcaaga tgagggcatt tatagcccta tcttatccat 10980
atggacaggt gccccccatg cgtccattta taggttctcc acaaaggtcg cattccattc 11040
ccagagctat gaacatctgc ttttctggga taggaatctt ggtgatgtga aacctctctg 11100
actgcacgtc cattcatagg ctctctgcag ggggaagcac atcacgcgct gttggctcat 11160
tctggcagtc cagcctggca ctgtccttac acaatcctgc atgcaatttt gtatttacaa 11220
taatcgggag catttcatct tttattccgt agcaatagtt tcaggggggg tctccctaca 11280
gtaaggaaca tgtttgttat tagagatttt attaaataag tcctctacta tattagccat 11340
gtgttttatt cagaatagcc atgaaaatta aacttctctg aagaattaat ttatgcctgt 11400
tgtaaaggaa ataactgtgg gttcaggacc agcaaaagca gataagtggt agaaaagaag 11460
ttatagactt ccatttttag atccacaaag tactgtttta attatactat agctacaata 11520
tataaacccc aaaatgacct tagcatttag taatggtagg aagaaggtca gaagatgtat 11580
aagtattagg gttttttctc aagtgaaaga tggtttagca gtgttaatat gatatgtgaa 11640
cacccctcgt tgtgtaataa ttaccatata cagagagtaa agggctgcaa ttagtatatt 11700
aagtcttata agcatgatgg tatgattaga tcaagagaat gacgctgtag ttatgaagac 11760

tactcctact agattgatgg tagggggcag ggcaaggttg gcaagactag ctaggagtca 11820
tcacatggct atcagggaga gaagtgtttg aaggccttgt gttagtagta tggtttggct 11880
gtgaactcgt ttgtagtttg agtttgcaag gcagaataat agggatgagg ttagtctgtg 11940
ggcagttatt agggaagttg cactggtaaa acttcagggg gtttgaatga gaaaagctat 12000
gataacaagt gccatgtggc ttatggaaaa gtaatgagtg attttagatc agtttggcac 12060
agacaaaagg agcttgttat gattatttct cataaggata gtaagaagga ggaataagct 12120
ataaactctg ttagggggtt aagaattaag gtgatttgta taatcctata gccatctaat 12180
tttaggagta ctgctgtgag gactattgac ctggcaattg cggcctctat gtgggctttt 12240
gggagtccta ggtggagtcc atagagagat atttttagta taaatgctat gatgcatgct 12300
aatcataaaa ggctattaaa ccaggaaatt gttagttctt gaagctgggt attggtctta 12360
aggtagactc gaaagtgctg ataaatttct caagcatcag tgtttgaata agcggagttt 12420
caaattttgg tctgctgtac attagctgtg agacactgaa aatgaatttg tcattctctg 12480
agctcagttt tttttttttt tttttttttt tttgagacag agtcttactc tgtagctcag 12540
gctggagtgc agtggtacaa tcttggctca ctgcaacctc catctcccag gtccccattc 12600
aagaaattct cctgcctcag tcccccaagt agctagcatt acaggcatgc accaccatgc 12660
tcagctaatt tttgtatttt tagtagagac gtggtatcac cttgttggcc aggctggtct 12720
tgaactcctg accttgtgat ccacctgcct tggcctccca aagtgttggg attacaggca 12780
ggagccacca cacctggccg tttgtttaaa atagagtaaa tagacctgct gaatatgttg 12840
atgtgagtat taattgtaat ctgcatagca attgtctgac cattgccttg aacatcacag 12900
gccatctgag tggcaagtat aatcatcatc atgtttctat ttaaaattca gaaatatttg 12960
aagcctgtgt ggctgaataa aagcatacaa atacaatgaa aatatcatgc taaatcaggc 13020
ttagcaaatg gacaaaatag taacttcgtt tgctgttatc tctgtctact ttcctagctc 13080
tcaaaggtct atggccctgt gttcactctg tattttggcc tgaaacccat agtggtgctg 13140
catggatatg aagcagtgaa ggaagccctg attgatcttg gagaggagtt ttctggaaga 13200
ggcattttcc cactggctga aagagctaac agaggatttg gtaggtgtgc atgtgcctgt 13260
ttcagcatct gtcttgggga tggggaggat ggaaaacaga gacttacaga gctcctcggg 13320
cagagcttgg cccatccaca tggctgccca gtgtcagctt cctctttctt gcctgggatc 13380
tccctcctag tttcgtttct cttcctgtta ggaattgttt tcagcaatgg aaagaaatgg 13440
aaggagatcc ggcgtttctc cctcatgacg ctgcggaatt ttgggatggg gaagaggagc 13500
attgaggacc gtgttcaaga ggaagcccgc tgccttgtgg aggagttgag aaaaaccaag 13560
ggtgggtgac cctactccat atcactgacc ttactggact actatcttct ctactgacat 13620
tcttggaaac atttcagggg tggccatatc tttcattatg agtcctggtt gttagctcat 13680
gtgaagcggg ggtttgaagc tgagagccaa gggaatttgc acatatttgt gctgtgtgtg 13740
tacaggcatg attgtgcgta cagtgtgggt ataaaaggtt catttaatcc catgttctcc 13800
tgaactttgc ttttttgctt tcaaataaga aatgatgaat atagattttg agttcatttt 13860

ttgaaagagt taaagagcag tgtttttccc attacctatt ccagaacatg tcaccagaga 13920
atacttgaca agtcaacatg gtgggaatgg ccctatcata cccatatgga gcatgaacca 13980
aatggcatgt gcttttattt aattggactg tgtttgtatg gtcagcctca ctgacttctc 14040
tggggtttct tttaggcccg tgcttgccat tctggccagt aatgacattc tacagttttt 14100
attgcttagg catatcttag tgcagttctc atcaattatt atttctctgt aaacacagca 14160
ttattttaaa aatagtatta attatttctt gttactgtat tgatttatat attttcagta 14220
aatacatcct gtagcataat tctgtgaaat acccaaatgt caatttataa aatgatttat 14280
ttaacaagat tttacttatt agtaataact ctgtaatctg cattccctat gtatgatttg 14340
gctctgtttc agttttgctt atctctttcc aaccatattt atgaaatttt ggcttagaaa 14400
tttatgttaa ttattttttt tccatggcca actctactca tctatgaagt tttacaatga 14460
atctgtttat cagcttggat accaaattac cttgttttta aattctgttt tcccaatgaa 14520
gttaaagact gaaaatcaga tttatctgtg aatgacacac acaaactaac agatttccaa 14580
ctgttcaatg cctggccatt cattcagagt acttttgatt aaagtcacta tttagggcct 14640
atagatatca ggggaaattc agcccctgat atttcaatgt gagtcctttt ctattctccc 14700
taagtgttgg ctggtctgag aaataaaggg aaagagtaca aaagagagaa attttaaagc 14760
tgggtgtcca ggggagacat cacatgtcgg caggttccgt gatgccccct aaaccacaaa 14820
accagcaagt tttcattagt gattttcaaa agcggaagga atgtgtgaat agggtgtgag 14880
tcacagagat cacatgcttc acaaggtaat aaaatatcac aaggcaaatg gaggcagggt 14940
gagatcacag gaccggagtg aaattaaaat tgctaatgaa gtttcaggca ctcattgtca 15000
ttgataacat ctcatcagga gacagggttt gagagaagac aaccagtctg accaaaattt 15060
attaggcagg aatttcctct tcctaataag cctgggagca ctatgggaga ccaggcctta 15120
tttcatccct tatctacaac tgtaaaagac agacgttctc aaaacagcca ttttagagac 15180
caccccttgg gaatgcattc ttctcaggga tgttccttgc tgagaaaaag aattcagtga 15240
tatttctcct acttgctttt gaaagaagtg aaatatggct ctgttctgct cagcccacag 15300
gcagccagac ttcaaggtta tctcacttgt tccctgaaca tcgctgttat cctgtttttt 15360
ttttcaaggt gcagatttca tattgtttaa acaatttgtg cagttaacac aatcatcaca 15420
gggtcctgag gtgacattcg tcctcagctt aggaagatga tggtattaag agattaaagt 15480
aaagacaggc ataggaaatc acaagagtat tgattgggga agtggtaagt gtccatgaaa 15540
ttttcacaat ttatgttcag agattgcagt aaagacaggt gtaagaaatt ataaaagaat 15600
gaatttgggg aactaataaa tgtctgtgaa atcttcacaa tttatcttct tctgccatgg 15660
cttcagctgg tccctccgtt cggggtccct gacttcctga aacatataga tgtgaatttg 15720
ggagctcttt aactataaag tttaatatct caaaataata agagctattt atgacaaacc 15780
catagccaat atcatattga atgggcaaaa gctggaagca ttccctttga aaaccagcac 15840
aagacaagga tgccctctct caccactctt attcaacata gtattggaag tcctggccag 15900

agcaatcagt caagggggta ttcaaatggg aagagaagaa gtcaaattgt ctctgtttgc 15960
aggtgacatg actgtatact tagaaacccc atcatctcag ccccaaaact gtttaagctg 16020
ataagcaatt tcagcaagac ctcaggatac aaaatcaatg tgcaaaaata acaagcattc 16080
ctataaacca ataatagaca agcagagagc caaatcatga gtggactccc attcacaatt 16140
gctacaaagg gaataaaatg cctacttaca caacttacaa gcgatgtgaa ggacctcttc 16200
aaggagaact acaaaccact tctcaaggaa ataagaggtg acacaaatgg aaaaaaattc 16260
cgtgctcatg aatagaaaga atcaatactg tgaaaatagc catactgccc aaagtaattc 16320
atagattcaa tgctataccc gtcaaactat cattgacttt cttcacagaa ctagaaaaga 16380
ataatttaaa tttcatatga agcaaaaaaa gagcctgtat agccaagaca atcctaagca 16440
aaaacaaagc tggaggcatc attctacctg acttcaaaca atactacaag gctacagtaa 16500
ccaaacagca tggtactggg aaaactggct agccatatgc agaaaacaga aactggaccc 16560
tttccttaca ccttatacaa aaattaactc aagatggatt aaacacttaa acataaaacc 16620
aaaaaccata aaaaccccag aagaaaacct aggcaatacc attcaggaca taggcatggg 16680
ccaagagttt gtgactaaaa cacaaaaagc aatggcaaca aaagccaaaa ttgacaaatg 16740
ggatctaatt aaactacagt gcttctgcac agccaaagaa actgcatcag agtcaacagg 16800
caacctacag aatgggagaa attttttgca atctatccat ctgacaaagg gctaatatcc 16860
agaatccaca aagaacttaa atttacaaga aaaaaagaaa tatccccatc aaaaatggac 16920
aaaggatatg aacagatgct tctcaaaaga agacatttat gcagccaaca aacatatgga 16980
aaaaagctta tcaacactgg tcattagaga atgcaaatca aaactacaat gagataccat 17040
ctcatgtcag ttagaatggt gatccttact aagtcaggaa acaacaggtg ttggtgagaa 17100
tgttgagaaa tatgaaccct cttacactgt tggtaggagt gtaaattagt tcaaccattg 17160
tggaagacag tgtggtgatt ccttaaggat ctagaactag aaattccact tgactcagca 17220
atcccattac tgggtatatg cccaaaggat tataaaccat tctactataa tgacacacgc 17280
ccatgtatgt ttatgtggca ccgttcacaa tagcaaagac ttggaaccaa cccaaatgcc 17340
catcaatgat gtactggata aagaaaatgt ggcacatata caccatggaa tactacacag 17400
ccataaaaaa ggatgagttc atgtcctttg caggaacatg gatgaagctg gaaatcatca 17460
tcctcagcaa ctaacacagg aacagaaaac caaactccac atgtttgcac tcataaatgg 17520
gagttgaaca atgagaacac atggacacag ggaggggaac atcacacatg ggacctgttg 17580
gggcacgggg ggccagcagg gggacagcat taggacaaat acctaatgcg tgtggggctt 17640
aaaacctaga tgacaggttc ataggtgcag caaaccacca tggcacatgt atacctctgt 17700
aacaaacctg cacgctctgc acatgtatcc tggaacttaa agtaaaataa aagaaaattt 17760
aaaaaatata aagtttatcg atcacttgat tataacaact tcatttcaga gatcctccac 17820
caaacaagaa aacttttcct ataatttaat tcatttagct attaaatgat acgctgggcc 17880
taatcaatat caaattaaat aggttaaatt tgcaggatga ttagagatga gggcatttag 17940
ttcctaaaga agaaaattca ttttaaaaaa ggtacagaag aaaaaaactc cacttatcaa 18000

tttacacttt cttgtttgcc ttacctggga tttatattta aaataaaagt ttttggccag 18060
gcgcggtggc tcatgcctgt aatcccacaa ctttgggagg ccaaggctgg tggatcatct 18120
gaggttggga gttcgagacc agcctgacca acatggagga acctgtctct actaaatata 18180
caaaattagc tggatgtggt ggcacatgcc tgtaatccca gctacctggg aagctcaggt 18240
ggagaatcac ttgaacctag gaagcagagg ttgtggtgag ctgagatcgt gccattacac 18300
tccagcccag gcaacaagag tgaaactcca tctcaaaaca aacaaataaa caaaaaacaa 18360
caacaacaaa aaacaacaac aaagttttca tttaattttt taacaaacta atctatgtta 18420
atttgatatt agtttgttaa cataacaaac taataatatg ttaggcatat tattcattta 18480
tatttgtttt ccttctcata aaacacattc tgctcttaca tttattttct taaatacatg 18540
aattcaccaa cagtctcttt ggttcctctc tactggttca acacatgact ctttcagcta 18600
ttcattatat ataaaaagct ttgaaatctc caactattct tgccctttcc atctcagtgc 18660
cttgctgtct actgactttg cagactgatg tgattccctc tgaaacatga attattaggt 18720
ttttagaaaa tgcctttttg ttctttccaa agtaaaagac aaataggctg ggaatgtaaa 18780
tttagcattt gaacaaccat tatttaacca gctaggttgt aatggtcaac tcaggattaa 18840
tgtaaaagtg aagtgttgat tttatgcatg ccgaactctt ttttgctgtt aagggaattt 18900
gtaggtaaga taatttctaa actactatta tctgttaaca aatacagtgt tttatatcta 18960
aagtttaata gtattttaaa ttgtttctaa ttatttagcc tcaccctgtg atcccacttt 19020
catcctgggc tgtgctccct gcaatgtgat ctgctccatt attttccata aacgttttga 19080
ttataaagat cagcaatttc ttaacttaat ggaaaagttg aatgaaaaca tcaagatttt 19140
gagcagcccc tggatccagg taaggccaag atttttgctt cctgagaaac cacttattct 19200
cttttttttc tgacaaatcc aaaattctac atggatcaag ctctgaagtg catttttgaa 19260
tactacagtc ttgcccagac agccttgggg tgaatatctg ggaaagacgg ccaagccctt 19320
tattttatgc atgggaaata aatgcctcaa tataggcctg atttctaagc ccattagctc 19380
cctcatcaat gttttttctg ctaaactcca aagccctgtt tctgtaaagt actttgttga 19440
cagccctaaa gcgtgctcat agcactccat ggatatccag gcactttgga ggctcccatt 19500
actcacaaga cttgtccttc aattaacact ttgtcgtatt atgtggcaga aatatcctaa 19560
tttaaaagac attatctcct tctagtaggg agaatatttg ggaccataga agctgccaag 19620
aaacactgaa tagggcaggg gtgtttgata tctcagttgg gatcctagct gatgagatag 19680
ctgggttagg aatgacaaaa ttattggttt tatggtgtat gaaccataaa cagacatcac 19740
acttataccc tgtgctgagc tggcatgttt tattctctgc ctaaataata attgtgtgat 19800
tttatagaag tcatttaact gctctggtgc acagttggaa tttgaagtat ctttgagccc 19860
ctcccacttc taaaatacta attccatttc agaggctgct tgatagaaat caatatagta 19920
gggactagct ttgtactatc aatcaggttg tccaaattct tttaacctat gctgtcatct 19980
acaaaacgtg aatgtagtaa ttcatgccat cttatatttc aagattatag agaagaattg 20040

taaaaagtaa cagaattaac ataaagatgc ttttatacta taaaaaggag gtctgagtct 20100
aggaaatgat tatcatctgg ttagaattga tcctctggtc agaattttct ttctcaaatc 20160
ttttataatc agagaattac tacatatgta caataaaaat ttccccatca agatatacaa 20220
tatattttat ttatatttat agctgtaatt tacaaccaga gcttggtata tggtatgtat 20280
gcttttatta aaatctttta atttaataaa ttattgtttt ctcttagatc tgcaataatt 20340
tttctcctat cattgattac ttcccgggaa ctcacaacaa attacttaaa aacgttgctt 20400
ttatgaaaag ttatattttg gaaaaagtaa aagaacacca agaatcaatg gacatgaaca 20460
accctcagga ctttattgat tgcttcctga tgaaaatgga gaaggtaaaa tgtaaacaaa 20520
agcttagtta tgtgactgct tgtgaatttg tgatttgttg actagttctg tgtttactaa 20580
ggatgtttaa ctggtcaatc agtaatgctt gagaagcact ttaagttttt attgtatgaa 20640
tgaataacaa aatagaccac tagctagact aataaagaag aaaaaagaga agaatcaaat 20700
agacacaata aaaatgataa aggggagatc accactgagc ccacagaaat ataaactacc 20760
atcagagaat agtataaaca cctctatgct aataaactag aaaatctaga agaaatggat 20820
aaattcatgg acacatacac tctcccaaga ctaaacaagg aagaagtcaa atccctgaat 20880
agaccaataa caagttctga agttgaggcg gtagttaata gcctaacaac caaaaaaagt 20940
ccaggaccag atggattcac agccaaattc taccaggggc acaaagagga gctgacacta 21000
ttccttctga aactattcca aataatagaa aaagagggac tcctccttaa ctcattttat 21060
gaggctagca tcatcctgat acccaaaccg ggcagagaca taacagaaaa agaacatttc 21120
aggccaatat ccctgatgaa caccaatgca aaaatccaca acaaaatact gccaaaccaa 21180
atccagcagc acatcaaaaa gcttatccac catgatcaag tagccttcat ccctgagatg 21240
caaggctgtt tcaatatttg caaatcaata aatgtaatcc atcacataaa aagaaccaat 21300
gacaaaaacc acatgattgt ctcaatagat gcagaaaagg cctttgataa agttcaatac 21360
ccttcatgct aagaattctc aataaactaa gtaatgatgg aacatatctc aaaatagtaa 21420
gagctgttta tgacaaaccc acagccaata tcatattgaa tgggcagaag ctggaaggat 21480
tccctttgaa aaccagcaca agacaaggat gccctctctc accactccta ttcaacatag 21540
tattggaagc tctggccagg gcagtcaggc aagagaaaga aatgaagtat tgaaatagga 21600
agagagaaag tcaaactgtc tctattcgca gatgacatga ttgtatattt agaaaacccc 21660
tttgtctcag cccaaaatct caagttgata agcaacttca gtatagtctc aggttataaa 21720
atcaatgtgc aaaaatcaca accatttcta tacaccaata atagacagac agccaaataa 21780
tgagtgaact cccactcaca attgctacaa agagaatata atacctagga cacaaacaaa 21840
tggaaaaaca ttccatgctc atggatagga agaatcaata tcatgaaaat ggccactgcc 21900
caaagtaatt tatagattca attctatccc catcaagcta tcattgcctt tcttcacaga 21960
actagaaaaa tctactttac attttatatg gaacgaaaga agagcccaca tagccaagaa 22020
aatctaagca aaaagaacaa aaagttcata atcactggga cattggagaa atgcaaatca 22080
aagtcccaat gaggtaccat ctcatgccag ttagaatggt gatcattaaa aagtcaggaa 22140

acaacagatg ctggagaaga tgtggagaaa taggaatgct tttacaccgt tagtgggagt 22200
gtaaattagt tcaaccatta tggaagacag tgtgctgatt cctcaaggat ctggaacaag 22260
aaataccatt tgacccagta atcctgttac tgggtatata cccaaaggat tataaatcat 22320
tctactataa agacacatgt gcacgtatgt ttattgcagt actattcaca atagcaaaga 22380
cttgggacca acccaaatgc tgatgaatga cagtctggat aaagaaaatg tggcacatat 22440
acaccatgga atacagtgct gccataaaaa aggatgagtt catgtcctct gcagggatat 22500
gggtgaagct ggaaaccatc atcctcagca aactaacaca ggaacagaaa accaaacatt 22560
gtatgttcta actcattaat gggagttgaa caatgagaac acatggacac atggagggga 22620
acatcaacac cggggcctgt cagggggtgg gggacttggg gagggatagc attagaagaa 22680
atacctaatg tggatgacag gttgatgggt gcagcaaacc accatggcgc gtgtatacct 22740
acgtaaaaaa cctgcatgtt ctgcacatgt atcccagaac ttaaagtata aatatataaa 22800
tctacccaag gagaaacaga atgcacattc caggtgaaat ggaatcaatt actgacaaat 22860
atgaaaacat aaaatatgcc ctgtgatgaa gatgggtgtc attattgcac acagtttatt 22920
tgagaatggg aaagaatgac ttaacctagg tgggtttcct gcattcctgc aaattgaaat 22980
aggccataaa atatttgcag tgagagtcag aggaggggtt tcagataggt tggaatgctc 23040
tacatggagg tgcagaggtg ggtgatggga aagagtaggt ggatggaaga gggaaaggaa 23100
gcctttattg agtgagaaca aaaatgattt gtacagataa cagttggaat ttagtgtcta 23160
tcagataagg cgtaaacatc aatgccgttg tcatcgcacc attgttgaaa acgctatttt 23220
ttcccctatt gaattgcctt ggcaaccttg tcaaaatcca attgactata aatgtgtgaa 23280
aggttttttt ctgtgtgctc tcaattgtac tccattgatc tatatgtctg tcgtaatccc 23340
tatagtgcac catcttaatt actgtagtat tgtattaata tcaagttatt atatcaaatc 23400
aaggagtgtg agtctttgtt gttctttttc aatacgtttt cactattctg ggtcccttga 23460
atcttcatat gaaatttaga atacacttgt caattttctc aaaggagcca atggggattt 23520
tgacacgatt gcattaaatc agtagatcag ggaattatgc catttttaaa attgaagtga 23580
aattcacaca atatacaatt aaccatttta aagtgtagac ttgagtggta tttagtgtat 23640
tcacaatgtt gtacaaccac cagctttctc tggtttcaaa atgtttttat tacttcacaa 23700
aaacatatca tattcattaa ataatcactc cattcctcca acccatcatg taattacctc 23760
taatcttctt tctgtttcta tggatttgcc tgttatggat atataaagga ctcacagcat 23820
atgtaacctg ttatgtctga gtcctttcac ttataatgtt ttggaggctc atctaagttg 23880
caacatgtat cagtacttca ttcttttttg tcgctgaaat atattttatt tttccatgca 23940
catatatcac aatttgttat tcatctgttt atggacattt gcattgtttc caacctttgg 24000
ctcttataga tagataatct tgctttgatc atttgtgtat gtttctttat gggcatattt 24060
tcatcattct tgggtatatt ctcaggagtt gaattgctgg gtcaaatggt aattttatgc 24120
ttatcttctt gtatataaga gccaatcttt tctacagtga atgtggcatt ttatattcct 24180

tccagcaatg tagaaggatt tctattttcc acatagttgc taatacttgt aactttctgt 24240
atttattgaa gccatcctag tgaatataga gtgttatctc attgtggttt tatttattta 24300
ttcatttatt tagagacaga gtcttactct gtcacccagg ctggagtgca gtggcatgat 24360
ctcagctccc tgcaacttcc acctcctggg tttacacaat cctcctgcct cagcccccag 24420
agtagctggg attacaggca tgtgtcacca tacctggtta attgttatat ttttggtaga 24480
gacgggtttt ctccactttg gccaggctgg tctcgaactc ttgacctcag gtgatctgcc 24540
catcttggcc tccctaagtg ctgggattac aggtgtgagc cactgcgccc agctctcatt 24600
gtggttttaa cttgtgttta atggccaatg attttgaact tcttttaata tattattacc 24660
catttgtata tcttctttgg ataaatgcca ttcaagtaca ttttctgttt aaattaagtt 24720
gactttattt ttctttttaa gctgttagaa tgatttatgt attcaaaaca ttaaactcct 24780
acatatacat aatatgaaga tatttcctcc cattctgttg gttgtcattt cacattctca 24840
attatattct tgctgcacaa agtttaattt tgatgaagtt tggtttatct atctttcctt 24900
ttgccactct ggtatcaaat ttataaatct attgccaaat atgaagtcat gaagatttac 24960
ccctacattt tattctaaga gttttatcgt tttagctctt atatttaagt ttttccatcc 25020
attttcagtt atatttttca tttggagtga agtaagggag atctcagctt cattcttctg 25080
catttcgctg tcctgttgtc tcatcaccat ttgttgaaga gactctttcc tcagattgaa 25140
tggtctaggc acccttgatg aaagtaaatt tgccatagat ctttaggttt atttcgtgat 25200
ttcagtttta ttccatttgt cttgatgtct atctttatgc tagtaccaca ctgttttgat 25260
tattacagtt tgtagtaagt ttgaagttgg aaactgagta ctcactgata cagaaattaa 25320
gtagaaatta cttaggcaaa tagtaagcat ttgggagtcc tcagtaaggt ttttctttat 25380
actggaaagc agccccaaat cattttctaa caaagagcag cttgtaaaat cgagctgcag 25440
acatacacaa ggaagctgga aacttgcacg agtgaaagct ggtagtaaag aactacctgt 25500
gaccaggcaa gttcaaaatg gcggctcctt ccccacccac tatgtaaatg tcacacctga 25560
ttaaaccaat ctctgggcca tacgtaaatc agacactgct tcctccagcc tccctatgca 25620
atctgctgtg gtccacctcc ttcccccttt tccgatgtcc ctctctttca caagaagctg 25680
cttttttctc tcctttcttc tattaaactt tctgctacat aacccactca gatgtgtccg 25740
tgtcctaaat ttttctgggg catgatgaca aacctgaggg tgtatatccc agacaacgta 25800
gctgcttcat caccatgtag ttgcatatta attgaacaat caactttccc atttctggaa 25860
aaaagaccat tggtattgtg gtagggattg cctgagtttt tagatcactt tgaggagtac 25920
tgccatctta acgatattaa gtcttccaat ccctgaacat gagatagttt tcaatttatg 25980
taggtattag atattctttt ttcttctgtt gcaagggtct ggtttagttt aaaaggcaag 26040
tttatcttaa atttttttat ttagttatga ccattgagga ttttaaaatg ataccatatg 26100
aaatgccttc ttttaaaaaa aatttcaact tttatttatt tatttattta tttattttta 26160
ttatacttta agttttaggg tacatgtgca ctatgtgcag gtctgttaca tatgtataca 26220
tgtgtcatgt tggtgtgctg catccatgaa ctcgtcattt acattaggta tatctcctaa 26280

tgctatccct ccccactctg ccaccccaca acaggcccca gtgtgatgtt ccccttcctg 26340
tgaccatgtg ttctcattgt tcaattccca cctatgagtg agaacatgcg gtgtttggtt 26400
ttttgtcctt gagatagttt gctgagagtg atggtttcca gcttcatcca tgtccctaca 26460
aaggacatga actcatcatt ttttatggct gcatagtatt ccatggtgta tatgtgccac 26520
attttcttaa tccagtctat cattgttgga catttggctt ggttccaagt cgttgctatt 26580
gtgaatagtg ccacaataaa catatgtgtg catgtgtctt tacagcagca tggtttataa 26640
tcctttgggt atatacccag taatgggatg gctgggtcaa atggtatttc tagttctaga 26700
tccctgagga atcaccacac cgacttccac aatggttgaa ctagtttaca gtcccaccaa 26760
cagtgtaaaa gtgttcctat ttctccacat cctctccagc accttttgtt tcctgacttt 26820
ttaatgatcg ccattctaac tggtgtgaga tggtatctca ttgtggtttt gatttgcatt 26880
tctctgatgg ccagtgatga tgagcatttt ttcatgtgtt ttttggctgc ataaatgtct 26940
tcttttgagc agtgtctgtt catatctttt gcccactttt tgatggggtt gtttgttttt 27000
tatcttgtaa atttgtttga gttctctgta gatcctggat attagccctt tgtcagacaa 27060
gagggttgca aaaatttctt cccattctgt aggttgcttg ttcactctga tggtagtttc 27120
ttttgctgtg cagaagctct ttagtttaac tagatcccat ttgtcatttt ggcttttgtt 27180
gccattgctt ttggtgtatg agacaagaag tccttgccca tgcctatgtc ctgaatagta 27240
ttgcctaggt tttcttctag ggtttttatg gttttaagtc taacatgtaa gtcttgaatc 27300
catcttgaat taatttttgt ataaggtgta aggaagggat ccagtttcag ctttctacat 27360
atggctagcc agttttccca gcaccactta ttaaatagtg aatcctttcc ccatttcttg 27420
tttttgtcag gtttgtcaaa gatcagatag ttgtagatat gtggccttat ttctgagggc 27480
tctgttctgt tccattggtc tacatctctg ttttggtacc agtaccatgc tgttttggtt 27540
actgtagcct tctagtatag tttgaagtca ggtagctttg ttcttttggc ttaggattga 27600
cttgacaatg cgggctcttt tttggttcca tatgaacttt aaagtagttt tttccaaatc 27660
tgtgaagaaa gtcattggta gcttgatggg gatggtattg aatctataaa ttaccttggg 27720
cagtatggcc attttcacga tattgattct tcctacccat gagcatggaa tgttcttcca 27780
tttgtttgta tcctctttta tttcattgaa cagtggtctc tagttgtcct tgaagaggtc 27840
attcacatcc cttgtaagtt ggattcctag gtattttatt ctctttgaag caattatgaa 27900
tgggagttca ctcatgattt ggctctctgt ttgtctgtta ttggtgtata agaatgcttg 27960
tgatttttgc acattgattt tgtatcctga gactttgctg aagttgctta tcagcttaag 28020
gagattttgg gctgagatga tggggttttc tagatataca atcatgtcat ctgcaaacag 28080
ggacaatttg acttcctctt ttccttattg aatacccttt atttctttct cttgcctgac 28140
tgccctggcc agaacttcca acactgtgtt gaataggagt ggtgagagag ggcatccctg 28200
acttgtgcca gttttcaaag ggattacttc cagtttttgt ccattcagta tgatattggc 28260
tgtgggtttg tcataaatag atcttattat tttgagatac gtcccatcaa tacctaattt 28320

attgagagtt tttagcatga agagctgttg aattttctca aaggcctttt ctgtatctat 28380
tgagataatc atgtggttct tgtcattgat tctgtttata tgctggatta catttattga 28440
tttgcctatg ttgtaccagc cttgcatccc agggatgaag cccacttgat catggtggat 28500
aagctttttg atgtgctgct ggattcggtt tgccagtatt ttattgagga tttttgcatt 28560
gatgttcatc agggatattg gtctaaaatt ctcttttttt gttgtgtctc tgcccggctt 28620
tggtatcagg atgatgctgg cctcataaaa tgagttaggg agtattccct ttttctattg 28680
attggaatag tttcagaagg aatggtacca gctcctcctt gtacctctgg tagaattcgg 28740
ctgtgaatcc gtgtggttct ggactttttt tggttggtaa gctattaatt attgcctcaa 28800
tttcagagcc tgttattggt ctattcagag attcaacttc ttccttgttt aatcttggga 28860
gagtgtattt gtcgaggaat ttatccattt cttccagatt ttctagttta tttttgtaca 28920
ggtgtttata gtattctctg atggtagttt gtatttctgt gggatcggtg atgatatccc 28980
cttcatcatt ttttattgcg tgtgttggat tcttctctct tttattcttt attagtcttg 29040
ctaacagtct accaattttg ttgatctttt aaaaaacctg ctcctaaatt cattgatttt 29100
ttgaagggtt ttttgtttcc atgtaattga gtttccattg tttccatgga atgcaaaaat 29160
tccatgtttt gagtgagttt cttaatcctg agttctagtt tgattgtgct atggtctgag 29220
agacagcttg ttataatttc tgttctttta catttgctga ggtgtgcttt acttccaact 29280
atgtggtcaa ttttggaata ggtgtggtgt ggcgaaaaga atgtatattc tgttgatttg 29340
gggtggagac ttctgcagat gtctattagg tctgcttggt gcagaactga gttcaattcc 29400
tggatatcct tgttaacttt ctctctcgtt gatctgtcta atgttgacag tggggtgtta 29460
atgtctccca ttattattgt gtgggagtct aagtctcttt gtaggtctct aagcacttgc 29520
tttattaatc tgggtgctcc gttattgggt gcatatatat ttaggatagt tagctgttct 29580
tgttgaattg atccctttac ctttatgtaa tggccttctt tgtccctttt gttctttgtt 29640
ggtttaaagt ctgttttatc atagagtagg attgcaacct ctgccttttt ttgttttcca 29700
tttgcttggt agatctttct ccatcccttt attgtgagcc tatgtgagtc tctgcatgtg 29760
agatgggttt tctgtgtaca gcacaccgat gggtcttgac tctttatcca atttgccact 29820
ctgtgtcttt taattggagc atttaaccca tttacattta acattaatat tgttatgtgt 29880
gaatttggtc ctgtcattat tatgttagct ggttattttg ctcattagtt gatgcagttt 29940
cttcctagcc ttgatgatct tacaatttgg catgcttttg ccatgggtgg taccagttgt 30000
tcctttccat gtttaatgct tccttcagga gctcttttag ggcaggtctg gtggtgacaa 30060
aatctctcag catttgcttg tgggtaaagg attttgtttc tccttcactt atgaagctta 30120
gtttggctgg atatgaaatt ctgggttgaa aattcttttc tttaagaatg ttgaatattg 30180
gcccccactc tcttctggct tgtagagttt ctgccgagag atcagctgtt aatctgatcg 30240
gcttcccttt gtgggtaacc cggcctttct ctctggctgc ccttaacatt ttttccttca 30300
tttcaacttt ggtgaatctg acaattatgt gtcttggagc tgctcttctc gaggagtatc 30360
tttgtggtgt tctctgtatt tcctgaattt gaatgttggc ctgccttgct agattgggga 30420

agttctcctg gatagtatcc tgcagagtgt tttccaactt ggttccattc tcctcgtcac 30480
tttcaggtac accaatcaga tgtagatttg gtcttttcac ataggcccat atttcttgga 30540
ggctgtgttc gtttcttttt attctttttt ctctaaactt ctcttctcac ttcatttcat 30600
tcatttcatc ttccatcact tatacccttt cttccagttg atcaaatcgg ctatagaggc 30660
ttatgcattc atcacgtagt tcttgtgcca tgattttcag ctccatcagg tcctttaagg 30720
acttctctgc attggttatt ctagttagcc attcatataa tcttttttca aggtttttta 30780
cttctttgcc ataggttcaa acttcctcct ttagctcaga ctagtttgat tgtctgaaga 30840
cttctctcaa ctcatcaaag tcattctcca tccaactctg ttccattgct ggtgaggagc 30900
tgtgttcctt tggaggagga gaggcactct gatttttaga atattcagtt tttctgctgt 30960
tttttccctg tctttgtggt ttcatctgcc tttggtcttt gatgatggtg acatacagat 31020
gggattttgg tgtggatgtc ctttctgttt gttagctttc cttctaacag tcaggaccct 31080
cagctgcagt ctgttggagt ttgccagagg tccactgcag accttgtttg cctgggtatc 31140
agcagcggag gctgcagaac agcggatatt ggtgaacagc aaatgttgct gcctgatcgt 31200
tcctctggaa gttttgtctc agaagagtac ccagccttgt gaggtgtcag tctgccccta 31260
atggggggtg cctcccagtt aggctacaag gggatcaggg acccacttga ggaggcagtc 31320
tgtccattct caggtctcaa gctttgtgct gggagaacca ctattctctc caaagctgtc 31380
agactgggac aattaagtct gcagaatttt ctgctgcctt ttgtttggct ataccctgac 31440
cccagaggtg gagtctacag aggcaggcag gcctccttga gctgcggtgg gctccacctg 31500
gttcgagctt cccagccgct ttgtttacct actcaagctt tggcaatggg gggcacccct 31560
cacccagcct cgctgctgcc ttgctgtttg atctcagact gctgtgttag gaatgagtga 31620
gcctcagtgg gcataagacc ctctgagcca ggcacgggat ataatctcct ggtttgccat 31680
ttgctaagac catcagaaaa gcacagtatt agggtggaag tgaccttatt ttccagatgc 31740
cttctgtcct tggctaagaa agggaattct ctgacccctt gcacttccca ggtgaggtga 31800
ttcctcaccc tgcttcagct caggctccat gcactgcacc cactgtcctg tacccactgt 31860
ctcacaatcc ccagtgagat gaacccagta cctcagttgg aaatgcaaaa atcattcatc 31920
ttctgcatcg ctcatgctgg gagctgtaga ctggagctgt tcctattcag ccatcttggc 31980
tccaaatgtt ctcaactttt attttgaagt gcacgttcat gtgtcttaca taggtatatt 32040
gtgtgatgct gaagtttagg gtacaaataa tgccatcaca caggtagtga gactagtacc 32100
caataggtag tttttcagcc cttgccttta tatctctcta ccctctatag taattccttg 32160
tgtatttttt ccatctttgt gtcccacttt tatatgagaa catgtggtat ttggatttct 32220
gttgctgcat taattccctc aagataatgg cttccagcta catctatgtt gctgcaaaga 32280
catgattttt tttaatggct gcatagcatt tcatggtgta tatacaccac attttgttta 32340
tgtgacacat gattggttgg ttctatgtct ttactattgt gaatagtgca ggaatggaca 32400
tactagcacg tgtcgttttg gtagaacaat ttgttttcct ctgggtacat actcagtagt 32460

ggaattgctg catcgaatgg tagttctgct tttagttctt tgagaaatct caaaactggt 32520
ttccatagtg gctgaactaa cttacattcc caccagtagt atataagtgt tctgttttct 32580
ctgcagtctc accaacatct gttatttttt ggctttttaa tcatatccat tttgattggc 32640
atgagatgat atctcattgt ggttttgatt tgcatttctc tgatgattag tgatgttgag 32700
tgttttctca tatgcttgtt ggccatgtgt gtgtcttctt tggaaggaca tatgtccttt 32760
gcccactttt taatggggtt acttgttttt taacttgttg aattgtctaa cttccttata 32820
gattctcgct attgaaacat tgttggattc acagtttgca aatattttgt cccattctgt 32880
aggttgtctg tttattctgt tgatagtttc tcttgctgtg cagaagctct ttaattaggt 32940
cccacttgtc aatattcatt ttggttacaa ttgcttttga ggagttattc ataagttctt 33000
tgctaaagcc tgtatccaga atgttatttt atagatattt ttccatcaga ttcttatagt 33060
ttgaggtctt aaatttagat cattaatcca tttcgagtta attattgtat atggttcaag 33120
gaagtgtttc agtttcattc tgcatatggc tagtcagtta tcctcattca atttactaaa 33180
tagagaattc tttcctcatt gattattttt tgtcaacttt gttgaagatc agatggctat 33240
aggtgtgtgg ctttatttct ggcttctcta cttctgttca atttgtgttt tgtaccagtt 33300
acatgctctc ttggttactg tagcttacag tatagtttga agtcaatgtg aagcttccca 33360
ctgttctttt tgtttaggat tgctttggat ttgggctctt ctttggttcc atatgaattt 33420
tagaataaaa taatattggt agtttgatag gaatagcatt gaatctgtag atagctttgg 33480
gcagtctagc cattttaatg atattgattg tttcaatcca taagcatgaa ctgtttttcc 33540
atttgtttgt gtcatctatg attttgttca tcagtatttt gtagctctcc ttgtagagac 33600
ctttttgccc cctttgttag ttgtattcct aggttttttc ttgtggctat tgtaaatggg 33660
attgagttct tgattggctt tcaggatgaa catttttggt gtttaaaaat gctacagatt 33720
tttgcatatt tattttgtgt actgaaatct cactgaagtt gtatgtcagt tccaggagtt 33780
tttggaaagt tctttagggt tttctaggtc tgaattgggt cttctaggtc tgagatgggt 33840
cttctacttc tactagtagg agatgggcct tgcaatcttg aagacagtag aaggatgggt 33900
cttattttta atccaacttc ctactctttg ccttttatgt ggagcattta gactttatct 33960
tcaatgttaa cactgatata tgaggttttg attctattgt gaagttgtta gctggttgtt 34020
ttgtagtttc tattgtgtga ttgctttata gagtctgtgg gctatatact taagtgtgtt 34080
ttgtggtagc aggtattgtt ctttcacttc catatttaga actcccttaa tgatctctta 34140
taaggctggt ctaatggtaa cagattttcc tggtagttac ttgtttggac aagattgtat 34200
ttcttccttg attttgaagc atagtttggc aaaatataaa attctttttt gtaattttta 34260
ttaaaatgaa tgttgaaaat aggctgagca tccttttagc ttgacaaggt tctctttgta 34320
tgtgatctga tttttttctc tagctgcctt taaaattatt ttttagcctt gactttggac 34380
agcctggtga gtaaatgcct tggtgatgtt aattttgtat agtgtctcac atatattccc 34440
tgggtttatt gtatttggat gtctaccttt ctagcaagtt tgggaaaatt ttctcaactt 34500
attagcccaa atgtattttc tagattgttt acttttcttt ctctctcagg aatgccaata 34560

attcctaggt ttactccttt taaataatca gatatttctt gaagactgtt catttattaa 34620
aattattttt tctttatttt tgtctgactt ggttagttca aaagactggt gttcaagctc 34680
tgaaattggt ccagtctatt gataaacgct tcaattgaat tctgaaattc cttaagtgac 34740
tctttcaatt ccagaagctt tgattgattt ctttttaaga tgtttacttc tcgctccatt 34800
tcctggactg ctttagtagt ttgtttgcat taattttcaa ccctgccttg gatcactgag 34860
cttacttgca gtccatattt tgaatagttt atctgtcatt tctgagttgc cagtttggtt 34920
agggttcatt gctggggagt tcatgtgatc ctttggaggt tttacgacat tcagacattt 34980
catggtgcca gaagtcttat gctggtttct tcttatttgg agaggctgcc gcttctgatt 35040
tttgaaatta tttttgtgca gatagaattg tttttctctc cctatttttg ttctttcctt 35100
tttctcttcc ccttcctatg ggatgtgact ataaagtatg ttgggtaggg cctttgactt 35160
ggattctata gctgtgttca cttctgtggg tagttttata ttggcctgtg cagtttgaca 35220
tacatgtcag tagatggcac ttatgagcaa aagccagctg tgactaggac aacttgatat 35280
agcacagctt gatccttgtt ttctggggga acttctctgt ttcctcagat aatctgttca 35340
tctgtggaat tcagagtagt ctgggctccc tgctgagccc cagggggata aagatcggtg 35400
tcactggact gggcagtcct gtctacaggt cccctaatgg caggcactag caccagcact 35460
gagagacaac tgatttttgc attgatcttg taccctgaaa ttatgctgaa atcatttatt 35520
agctctactt attttctatc tgtattgcat gagatttttc tatatagaga gaatcatgtt 35580
tcagtcaaat acacatgatt tgaatttttc ctttcctatt tgaatgtgtt ttatttcatt 35640
tcttgcctaa ttgctctggc tagaacttcc aatactatgt ttaatagtag taaaaatgga 35700
catccttgtt ttttttcttt tctttctgat tttaagttac aagttttagt ctttcacaat 35760
ttagtatgct ttagctttgc agttttcata aatatgcttt attatgataa aggagtttta 35820
tttcatttat agttttctga gtgtttttgt tatgatgggt attaaaagtt gtcaattttt 35880
ttttctgcat ctaatgagat gaacatgatt ttctctttta ttctgatatg gtgtagtgca 35940
ctgattgact ttcttgttga accacttttg aatttctggg ataaatctca cttgcttatt 36000
gtgagattta tttttataat gtttattatt ataatttttt aattttgtaa aaaattgttg 36060
caaaatacac ataacataaa agtttctgta ttaatcattt taagtgcata gtattttggt 36120
attaattaca ttcacattgt tgtgcaaatg tcactgccat acatctgtag aattcttttc 36180
atcttgcaaa acagaaactt tatacccatt taataataac tcttggccgg gcattgtaga 36240
tcatgtctgt aatcccagca ctttgggagg ttgaggtggg cagattggat gagctcagga 36300
gttcgagact agcctgggta acatggcaaa accctgtctc taccaacaaa gcaatcaaca 36360
aacaaaatga aagtaacata atttcctcaa ctgtgcaaac taattcatat taataattcc 36420
atgaaatgtg tccatagtaa cacaagagca gaagatgggc cttgtaaagg tggcccacca 36480
cattatttga ggtctttgtc agtcttatct gccagatact gtgtggacta actgcagtta 36540
aaattcccat tcccttatca cttgccctga aatttatctg aaatcaaagg gaatgggttg 36600

cccaaagaga agtgtccaga gacgtggatg atgcgacaag aaatgttgta gaaccacacc 36660
cccacctcaa atgataactc tacatttctc ccttccacca ccccatggca accaacattc 36720
tactttgtgt ctctataaaa tcgagtatgc tgggtacctc ataaaagtgg aatcattaca 36780
tatttgcctt ttgtgactgg tttatttcac ttagcctaat gttatgttct cagggttcat 36840
tcatgttttt gcatgtgtca gatttttctt cctttctaag ggtgaataat attccgttgt 36900
atgtataaac caagtttgct tacccattta tctgttgatg ggcacttgga ttactttcac 36960
cttttgactt tttgagtaat gctattatga atatgggtaa aaaaatatct ctttgatgtg 37020
ctgctttcaa ttcttttggg tataaataac ccaaaagaat aacccaccac gcctggttaa 37080
ttttttgtat ctttagtaga aactgagttt caccatgttg gccaggctgg tctcaaactc 37140
ctgaccctgt gatccaccca cctcagcctc ccaaagtgct ggaattatag gtgtgagcca 37200
cgtcgcccgg cctacattta ttgatttgca tatgttgaac caggcttgca tcccagggat 37260
gaagccaact tgatcgtggt ggataagctt tttgatgtgc tgctggattc agtttaccag 37320
tattttgttg aggattttcg catggatgtt catcagggat attggcctga aattttcttt 37380
tttttgtttt atgtctgcca agttttggta tcaggatgat tcttccctta taaaatgagt 37440
taggaaagag tgcgtctttt tctactgttt ggagtagttt cagaaggaat ggtaccagct 37500
cctctttgta cctctggtag aattcggctg tgaatccttc aggtcctggg cttttttttt 37560
gcttggtgag ctattccttg ctgcctcaat ttcagaactt gttattggtc tcttcacgga 37620
ttcgacttct tcctggtttg gtcttgggag ggtgtgtcta ggaatttatc catttcttct 37680
agattttcta gtttatttgc atagaggtgt ttatagtatt ctctgatggt agtttgtatt 37740
cctgtaggat cagtggtgat atccccttta tcattttttt gttctctcta tttgattctt 37800
ctttcttttc ttctttatta gtctggctag cagcctattt tgttaatctt ttcagaaaat 37860
tagctcctgt attctttgat attttgaagg gattttcctg tctctatctc cttcaattct 37920
gctctgatct tagttatttt ttgtcttctg ctaggttttg aatttgtttg ctcttggttt 37980
tctagttctt ttaaatgtgt tgttagggtg ttgattttag atctttcctg ctttctcctc 38040
tggacattta gtgctataat tttccctcta aacactgctt tagctgtgtc ttagagattc 38100
tggtacactg tgtccttgtt ctcattggtt tcaaagaaca tctttatttt gccttcattt 38160
tgtcatttac ccagtagtca ttcagagcag gttgttcagt ttccatgtag ttgtgcagtt 38220
ttgagtgagt ttcttaatcc tgagttttaa tttgatggca ctgtggtcta ataggttgtt 38280
tgctttaatt tctgttcttt tgtatttgct gatgggtgtt ttacttccaa ttatgtggtc 38340
aattttagaa taagtgtgaa gtggtgctga gaagaatgca tattctgttg atttagggtg 38400
gagagttctt tagatgtcta ttatgcccgc ttggtccaga gcttagttca agtcctggag 38460
atcctagtta accttctgtc tcattgatct gtctaatatt gacagtgggg tgttaaagcc 38520
tccatctatt attgtgtggg agcctaagcc tctttgtagg tctttaagaa cttgctttat 38580
gaatctgggt gctcctgtat tgggtgtgta tctatttagg atagttagct gttctcgttg 38640
cattgatccc tttaacatta tatgatacca cccctttttt taatctttgt tggtttaaag 38700

tgcgttttat cagagactag gattgcaacc cctgctttgt tttgctttcc atttgcttgg 38760
taaatattcc tccatccctt tattttgagc ctatgtgtgt cttttcacgt gagatgggtc 38820
tcctgaatac agcacattga tgggtcttga ctctatccaa tttgccagtc tgtgtctttt 38880
aattggggca tttagcctgt ttacatttaa ggttaatatt gtgtgtggat ttaatcctgc 38940
cattatgatg ctagctgatt attttgcctt ttagttgatg cagttttttt tatagtgttg 39000
acggtcttta caaaatttgg tatgtttttg cagtggctgt tcccagttga tcctttccat 39060
atttagtgtt tccttcagga gctcttgtaa ggcaggcttg gtgatgacaa aatccccttg 39120
tggtgatggg cttccctttg tggtaacccg acctttcttt ctggcttccc ttaacatttt 39180
tcctttcttt taaccttggt gaatctgaca attatgtgtc ttggggttgc ccttcttgag 39240
gagtatcatt gtgggtgttt tctgtatttc ctgaatttga atgttggtct gtcttgctag 39300
attgtggaag ttctcctgga taatatcctg aagaatgttt tacaacttgg ttccattctc 39360
cccatcactt tcaggtacac caatcaaacg tagatttggt cttttcacat agcgccacat 39420
ttcttggagg ctatgttcat ttctttttat tcttattttt taactttttt cttcacattt 39480
tatttcatta agttgacctt gaatctctga tatcctttct tctgctttat caatttggct 39540
attgatattt atttatgctt cacaaagttc tcattctgtg ttcttcagct ccatcaggtc 39600
actcatgttc ttctctagtt agcaattcgt ctaacatttt ttcaaggttc ttagcttctt 39660
tgcaatgggt tagaacatgc tcctttagct cagtggagtt tgttattaca caccttctga 39720
agcctacttc tgtcaattca tcaaattctt tctctgtcca gttttgtccc cttgctggca 39780
aggagttgtg attctttgga ggagaagaga tgttctggtt ttttaaattt tcagcctttt 39840
tgtgctgttt ttttttttct catatttgtg gatttatcta cctcttgtct ttgatgttgg 39900
tgacctttga atgggttttt tgcgtggaca tcctttttgt tgatgttgat gctattcctt 39960
tctgtttgct agtttttttc ctaacagtga ggcccttctg ctgcaggtct gctggagttt 40020
gcttgaggtc cactccaaac cttgtttgac tgggtatcac cagaggaggc cacagaacag 40080
caaagattgc tgtgtgttta ttcctctgga agcttcatcc tagggggaca cttgccagat 40140
gccagccaaa gctcttttgt atgaggtgtc tgttgacccc tgctgggagg tctgctgttc 40200
tctttagagc cagcaggcag gaacatttaa gtctgccgaa gctgcaccca ctgccacccc 40260
ttcttccagg ttctcagtac agtgagatgg gagttttatc tataagcccc tgactgggac 40320
tgttgcctgt ctttcacaga tgcaactgct cagagagtag gaaactagag gggcagtatg 40380
gctacagtgg ctttgcggag ctgaagtgga ctccacctag tcctagcttc ctggaggctt 40440
tgtttacact gtgaggggaa aaccacctac tcaagcctca acaatggcag actcccctca 40500
cccccccacc ccccccaagc tcgaacatcc ccagtcaact tcagactgct gtgctggcaa 40560
acagaagttc aagccagtgg atcttagctt gctgggctcc atgggggtga gatcccctga 40620
gctagatctc ttggctccct ggcttcagtc ctctttccag gggactgaat ggttctgact 40680
tgctggtgtt acagcaccac tggggcataa aaaaacccct ccagctatct tggtgtctgc 40740

ccagatgacc acccagtttt gtgcttgaaa cccagggccc tggtggtgta ggctctgaag 40800
ggaatcttct ggtctgtggg ttgtgaagac catggaaaaa ctgcagtatt tgggccagag 40860
tgcaccgttc ttcacagcac attccctcaa ggctttcctt ggctatggga gggagttcac 40920
caatcccttg ggcttccaag gtgagacaac attcctccct gcttcagttt agcctccatg 40980
cactgcacct actgtctaaa cattcccagt gagatgagcc aggtacctca gttggaaatg 41040
tagaaatcac ctaccttctt cattgatctc gctgggagct gctgactgga gcttttctta 41100
ttcagtcatc ttgccagcca ccctcctgat tgagtttctt taaggtaatt attttgaatt 41160
ccttttttga atatttgtgg attttttttc attgaggtct gttatttgag agttattatg 41220
tccctttggt ggtgccatat ttctttattt ttcatgttgt gtctctgaat ttatgtctat 41280
gcatgtgatg gaacaattgc tattccaaaa tctctagagt gaatttcata gagaaagact 41340
tttatctgaa gttgagtgtt tgtgtgcagg tgacgaagag tgggtaactc agtttttgat 41400
agatgcattg tgatagtatc tgtgtagttt ctctagctat ggccaatatc agcaataatt 41460
ttgggtgcct cagtggccta ggctgtagaa gtttgcagca gtatcaggag cagtgtaggt 41520
tgttaatgtc cttagtgtca aagtatttgg gcgtcctcct atttttattt tcctaaaatt 41580
ggggtgactt agcctaaggg attccttttg gtgtcatctc tgacatggca ttaactcagc 41640
ggcattggtg ctgggttaca agtacagatc cttgaagttg tcatagaagc atcattctag 41700
actcagggtc ttttaatcat gtattgtgac acctgggtct tgcggtgcag gttcactctc 41760
tgtggcagtg ttgaatgcag attacccaca gaaccaaggt ctatgactct agcacacctt 41820
agcagctgaa gcccacagac tgagttgtgg ctgtgaatct gtttctgagg gtgaggtgca 41880
ggaaaatgcc gtggcctgcc tctgatgtag aaggggtgct ctttagattt gagcccagca 41940
agtagaatat ggctactatc attaataaga taggatatac taccttatta atgctaagta 42000
accaagctca agtcaagaga tggaaatgtt aatactggtt tgtgactgca ctgttcttct 42060
tacttaagag taattttcaa catggcaagc ttagaaaact gtgtgaagct gatgttacca 42120
agggagagga ctgtctttgt ctacacatgt tgagagaatc tagtctattt cagtggtgtt 42180
gattacacaa tattcaacat gacacacatc acttggagac tctaaaagca tcattaattt 42240
attgctgctg gccttggggc tggttaaaaa gtaatacgtt ttttatattg atgatctctt 42300
tcaactaagt attccttatc cattgctttc tttctcccaa cttccaaatt atgagaactc 42360
cagtctctaa gccttggcat atgcagtatc cttactctct cctttatccc ctgtggcaat 42420
taatcccttt attctccatc ttcatcattg ttctttctct tcactttgta tatttttgtt 42480
tattcctgca ctactgcacc ctagaacaag ttgtctgttg cctcccatct agattataat 42540
agtgtgggcc catcttgtca ttgtaaaccc attctcttcc tctataattt atcttaggtt 42600
ttgctgtcat attgcttttt taatatttgt ttttacaaaa tttcaacttt ataatgaatc 42660
tgggggcaat attgagtcca tgtacctttc tgacagaaaa atataattgc agactcttga 42720
ttcaggctca cagtgtctaa tgatatggcc ccaatttacg ttttctatta atttagattg 42780
tggttgtcta caggtaataa tggctttaac acataatgtc tttatttatc tcaaatacag 42840

ggattctctt ggtggggcaa tctaggataa tacagcagcc ccaaagcagc agagagtcaa 42900
gtgccttcct ttttcccact aaatttagta tacagacctt cattttgatg attaaatccc 42960
aggatggggt ctactctacc tcctacttca catttgtgtt ttgagtatga aagaggaaat 43020
agaggaaatg gacctagaga ccttctgctt atatatttta gcaaaaaaaa atatgctgtg 43080
tgactcagct agctgcaaag agcctgatga atggaatttt taggcaagca tggaataagg 43140
gagtaggaaa taaagtttgg gcaagttggt ctacagcctc tgctatacaa gcagtatttt 43200
ttttctagta ctgtactttc cagtttctat gttggtaact atataactat gtgaataatt 43260
ttgaattcac tgtaatcaaa tatgctggta aataatttgt cagataattg catcaaatca 43320
ttcctaggaa aagcacaacc aaccatctga atttactatt gaaagcttgg aaaacactgc 43380
agttgacttg tttggagctg ggacagagac gacaagcaca accctgagat atgctctcct 43440
tctcctgctg aagcacccag aggtcacagg tatgatcaga gatgataagt taattaattt 43500
tcagaaaaga ttttgggaag gtgttgctag tgtcctcctt cctgtttctc ttagagaagc 43560
ttcattattt aaacttttgt gcttccagct gtaatctgtt tcaaactaat ggtgattaca 43620
atgggatatc ttggcctggc atggtggctc acacctgtaa tcccagcact ttgggaggct 43680
gaggtgggtg gatcacttga ggctaggagt tcaagaccag cctggccaac atggccaaat 43740
cccatctgta gtaaaaatac aaaacttagc caggcatggt ggcacatggc tgtaatccca 43800
gctactcgag gggctgaggc actggaatca attgaaccca ggtggtggag gttgtagtga 43860
gccaagattt tgccactata ctccagtctg ggcaacagag tgagactctg tctcagaaag 43920
aaaaaaaaag aaaaaaagaa aaagaaaaga taaaaggggc atctttgcac agtagaggaa 43980
gataactgag agaaatgaag acagcatggc agtagcaaaa gcagtagaac tctggtccaa 44040
tgtgtctgga tttatggcag gaagagacaa gataaattgg cctgggattg catgttggtt 44100
ttattatgag aaggccattt taaatagcaa gtatattctt caagataact tttctcattc 44160
tcaaaatttc aggttcgaat gctggagtag ggaaacttga aactctcctt attgaaggat 44220
aaatggtaat cctaaaaatg tagtgatctg cctgagaaaa ttcctagtcc aataagtact 44280
ctaaaaagtt agattcataa gaaaggtgat tctgtttact agaagaggta tataaagaat 44340
gttccattta ggcagaatat gtacctgaga cagtttccat gaaactgttg ttgggcaaaa 44400
taagattttt ttgaggaagt caataatttt atcttttatg aacaacttta ctttagaaat 44460
ttacttttaa ggacttttgg ttatgctgca gataagaaat attctttttt tctcctatgt 44520
cagtatcccc cattgaaatg acaataacct aattataaat aagaattagg cttttttttg 44580
aacagttact agcctataga gttctagaag attttttgtc aaattttatt atagattaaa 44640
gggtacaaat acaggtttgt tacataggta agttgtgtaa cactgaggct tggggtccca 44700
gctattccat cacactgaca gtaaacatat tacccaacag atggttcttc agcccacatc 44760
tcttctctcc ctcccccatc tagtgatcct cagtgtctat tgttcccatc tttatgttct 44820
tgtgtattca atgcttacct cccacttata agcaagaaca tgtggtattt ggttttctgt 44880

tcttgtatta gattgcttag gataatggcc tcctgcttca tctataatgc tgcaaaagac 44940
ataacttttg ttctttctta tggctgcata acattccatt atgtatgtac tagtctgttc 45000
tcatgctgct aataaagaca tacctgagac agggtaactc ataaaggaaa gaggtttaat 45060
ggactgtcaa ttccacacgg ctggaaaggc ctcacaataa gtctctgata gagacttatt 45120
cactatcaca agaatggcat gggaaagtca ggcctccatg attaaattac ctcacattga 45180
gtacctcccg tgacgtgtgg gaattatggg agctacaatt caagatgaga ttttggtggg 45240
gacacagcct aaccatatca gtgtatattt ggtacatttt ccttatacaa tccactgtcg 45300
atgggcacat aagttgattc catttctttg ctatcgtgaa tagcactaca tattggggtg 45360
aacatattgg ggtgtgtgtc ttattgatag aatgaatatt ttgggggcat atactccata 45420
gtgggattgc tgggtcaact ggtagttcta ttttaagttc tttcagaaat atccaaactg 45480
gtttccacag tggctgaact agtttgcata cccaccaaca gtgtaaaagc atttccattt 45540
ctctgcaacc tcaccagcat ctgttgtttt tggacttttt aatattaata attgctattc 45600
tgactggtgt gagatggtac ctcattgtga ttttgatttg catttctctg atgattagtg 45660
atgttgagca ttttttcata agtttgttgg ctgcttgtat gtcatctatt gagaagtggc 45720
tgttcatgtc ttttgcccaa ttttaattga ttttttttgt tttttttttt tgcttgttga 45780
tttgtttaag ttttttatag attttgtctg ttgatacata tttagatttt ggatattaga 45840
catttaggtc ctttaaggta ggaagagtac aggaaatagt caagaagact ttaacaaatt 45900
tctaaaaaca gagagcatat agaggaatga tatatctgta cagagagggt aaacttataa 45960
ccaaaaggtc agaagagaaa aatttatcaa ggatgaaaag ttaatcagtg tcccttaaat 46020
cctcccaata ttggtatttg gaagtaccga gggtcaaaga tggtggagaa tgaggctgaa 46080
aaaggaggct ctccctgcct acgtttcctc cacaactctt ttcatcaggt aactgatttc 46140
cttccacaag aagaaggagg ctcactctct agagaaactg agcttaagaa gccctgtact 46200
cagagacaca cagcagtgcc agaaaaggca tgaggtgtag gctagaaaga cagattaggt 46260
acacatccac tccttgaatt cagagactct tagctctttt attcatcttc cttcctaaaa 46320
ttcagcagct agctctatcc tacagaaagg gaactgatct agagaaatgg catttttctt 46380
ttgccatctt aacagacaat tctcttcaat gtccaacaga gtgaatgaaa tgaaaacata 46440
tactgctaga tacagggtta tggaatttca ggacatgaga attaaaggga aaatcacaaa 46500
aactttcaga gctgtaccag gttatattga aagaattaga attcagaatt ctcaagaaga 46560
actattaata ttatcttgta tattaataaa aatagtgttt tcagatgtgc atgtttgcca 46620
aaaattttta ccattttccc tttctcagaa aaatactgaa gaatgttctt cattcaaatg 46680
cctgaaacct tgttagagaa agtcctgagc tccagcaatc aggaaacctc gtgtaggaga 46740
atgagagtgt cagggaggct gcctagcacc agacctagaa aacaactact tcagaacaga 46800
cctgggggat aattcagaag tgagatgaga cagcaagaac aaaagagatg tgacagattt 46860
tatgagagtt tccaataact aaaaaggagt ttcaaacatc tgctggaaca tttgtaaata 46920
attctaactt gggaaatgga aaattacaaa ttgaaataaa gagacattgt taagcccagg 46980

aaagtacaaa aataaaattt aatcatagtt gtcttaccag agaaccatat ttgtgtagtc 47040
agaatgatgt aagcaatgaa tactgatatc ataaaaattg tcatctaaat atattctgga 47100
aaaagcagtg tggaaaagtg agtgtgtcta tctgagggtg tgttcaaata tgaggggaag 47160
atggagatta aaattctcat ctctcttaat aaaaaattat taaatataat ctacaaaaag 47220
ataagtaaga attagaaaca gagtatttaa aaatgtgaat gtatgttcag aagaagctgc 47280
tagaatgttc aaaatggtag atttggaaag tgtgagaatg aagagaatac cattgtgatt 47340
tgtgacccct cccacacaca gatgcaccaa tacagtcata tatatgcata aatctgatag 47400
tataatttaa cgtttaagca acatatgtta ttttgatatg aaattaatct gtttttcaaa 47460
gacagtaatt tcatgactaa attatatgct tactttatgt tttcaaacat tttattattt 47520
ttatttgtat taattatcat atatactaat gtatatgtat atacatatct tttaattttt 47580
tttaattttt atgggtacaa agtaggtata tatcaatatg tttgggggta catgagatgt 47640
tttgatacag gcatgcaatg tgaaataagc atgtcatgga gaatggggta tctattccct 47700
caagcattta ttctttgagt tacaaacaat tcaattacac tctttaagtt attttaaaac 47760
gtacaattaa gttattattg actatagtca cccttttgtg ctatcatata gtagatctta 47820
ttcatctcca cctcccctcc ccccacacta ccctttcgag tctctggtaa ccatctttct 47880
acactttatg tccattagtt caattctttt atttttagat cccacaaata agtgagaaca 47940
tgctatgttc gtctttctgt gcctggctta tttcacttaa cgtaatgatc tccagttccc 48000
ttcatgttgt tgcaaatgac tgaatctcat tctttttatg gctgaataga actacattgc 48060
gtatatgtac tacattttct ttatacattt gtctgttgat gcatatttag gttgtttcaa 48120
agtcttagct attgtaaaca gtgctgcaac aaacaagagt gcagatacct cttcgatata 48180
cctatttcct ttcttttggg tatataccaa gcagtagggt tgttggatca tatggtcgct 48240
ttatttttag gattttgagg aacatttaaa cagctctcca tagaggtcac actaatttgc 48300
attcccacca acactgtacg agggtccact tatctccaca tcctcactag catttgttat 48360
tgcctgtctt ttggatataa gccattttaa ctggagtgag atgacatctc attgtagttt 48420
tgatttatat ttctctgagg atcagtggtg ttgaccacct ttttgtatgc ctgtaaccca 48480
tttatatatc ttcttttgag aaacatctat tcaaatcttt tgcccacttt tggattggat 48540
tattagattt tttttctata gagttgcttg aatttcttat atattctgat tactaatctc 48600
ttgtcagatg aatagtttgc aaatatttct ctccattctg tggattgtct tttccctttg 48660
ttgattgtat ctttggctgt gcagaagctt tttaacttga tgtgatccca tttgttcatg 48720
tttgctttga atgcctgtgc ttgtagggta ttgctcgaaa aatttttgcc tagaccaatg 48780
tcctggagat tttccctagt gttttcctat agtagtttca cagtttgagg tcttagactt 48840
aagtctttaa tccattttga gttgattttt gtatatggca agggatatga gtccagtttc 48900
attcttctct gtaggatatt aaattttccc agcacattta ttgaagagac tgtctttccc 48960
ccagtgtacg ttcttggcac ctgtgttagt ctgttctcat gcctctaata aagccataca 49020

caagactggg taatttataa aggaaagaag tttaatggac tcacagttcc acatggctgg 49080
agaggcctca caatcatggc agaagatgaa tgaggagaaa agtcatgtct tacgtggtag 49140
caggcagata gctttgcagg ggaactcaca tttataaaac catcagatgt tgtgagattt 49200
agtcactatc atgagaaaaa tatggaggaa accaccccca tgattcaatg atccctacct 49260
ggcctccccc ttgacttgtg gggattattt caattcaaga tgagatttgg ggtggggaca 49320
cagccaaacc atatcagccc ctttgtcaaa aatgagttaa ctctagatac gtgaacttgt 49380
ttctgaattc tctattctgt ttcattggtc catgtgtttg tttttatgtc agtaccatgc 49440
tgttttagtt agtatagctc tgtagtttaa tttgaagtga gataatgtga ttcctccagt 49500
tttgttcctt ttgcttagga tagttttgac tattctgggt cttttgtggg tccatataca 49560
ttttaggatt tattttttct atttctgtga agaatgccat tgatattttg ataggaattt 49620
tattgaacct gtagattgtt ttgatagtat gggcacttta acaatattga tttttccaat 49680
ccatgaacat gaaatatttt tctgtttgtt gggtcctctt aaatttcttt cctcagtgtt 49740
ttatagtttt cattatagag atctttcaat tctttggtta aattaatcct aggtatttaa 49800
aattatgtat ggctattttc aatgggatta ttttttaaat ttctttttct tttttttcac 49860
tgttggcata taacaacact actgattttt atgttgattg tgtatccttc aactttactg 49920
aatttgttca tcagttctaa taattatctt gtagaatctt tagtttttcc tactataagg 49980
ttatatcatc tgcacacaag gatattttga cttcttcctt tccaattttg atagccttta 50040
tatctttctc ttgtctgatt gctcttgcta ggtcatccag tactatgatg aataacaatg 50100
gtgacaatgg gcatccttgt tgtgttcctg atcttacagg aaagacttcc tattttctcc 50160
attcaggata gtactagttg tgggtctgtc atatagcttt tttatgttga ggtatgttcc 50220
ttctatctca agtttttttt atgaattttc atcaagaagg gatgttaaat tttatcaaat 50280
actttttcag catcaactga aatgatcata tggcttttat ccttcattct gttgatatga 50340
tgtatcacac tgcttgattt gcacatgttg aaccattctt gcatccaagg gataaatccc 50400
acttggtcat tgtgaatgac ctttttattg tattgttgaa tttggtttgc tagtgttttg 50460
ttgaagattt ttgcattagt gttcatcagg tgtattggcc tgtagttttt tttttttttt 50520
tttttttttt tgatgtgtct tagtctgatt ttggtatcag ggtaatactg gcctcataga 50580
atgagtttgg aagtattctc ttctccttta ttttttggaa tagtttgagt agggtcggta 50640
ttagttcttc tttaaatgtt tggtaaaatt cagcagtgaa gctagccagt cttgggattt 50700
tctctactgg gatgatttta tttctcgttt gatctttgtg ctggttattg catgttcaga 50760
ttttggattt cttcctggtt caatcttgat gggtcatatt tgtgtaggga tttgtccatt 50820
tcttctagat tttccaattt attggcatat agttgctcat agtaaccaat aattattctt 50880
tgaatttctg cagtatcagt tctaatgcct cctttttaat tttttattta tctgaatcta 50940
ctcttttttt ttttcttagt ctggctaacg gtttgtcaat tttgtttaac ttctcaaaaa 51000
agcaactttt tgtttcattg atcttttgtt ttgttttctt cacttaaatt ttatttattt 51060
ctgctctgat ctttattatt ccttttcttc tactaatttt ggttttaatt tgcttttgct 51120

tttctaattc tttaggatgc atcattaaat tgtttgtttg tcaattttct ccatttttga 51180
tataagctct tgtagctata aacttccctc ttagtactgg tcttgctgta acccctaggt 51240
tttggtatgt tgtgtttcca ttatcatttg tttcaagaaa tttttcaatt ttctttttaa 51300
tctcttcatg ggtccactgt tcatttatga gcatattttt aaatttccat ttatttgtgt 51360
agtttccaaa attcctcttg ttattggttg ctggttttat tacattgtgg tcagagaaga 51420
tgcttgatat tatttcagtt tctttgaata ttttaagact tgttttgtga cctaacatac 51480
ggtcaattct tgataacaat ccatgtgctg tggaaaagaa tgtgtattct gtagcagttg 51540
gataaaatat cctgcaaata tctatgagat ccatttgatc tatagtgcag atgaatttca 51600
atgtttcctt gttgattttc tatctggatg acctgtccaa tgctgaaagt ggggtgttga 51660
agtctccagg tattattata ttggggccta tctctctcta gttctaatta tatgtctttt 51720
atatatctgg gtgctgcatt attggttgca tatatattta aacttgttcc atcttcttgc 51780
caagctgacc actttatcac caatagtgat cttctttgtg tctccttatg gtttttgttt 51840
tgaaatctac tttgtctgtt ttaaatatag taactcatgc tctttttttt catttccatt 51900
ggcaggtact gtctcattca attcctttat tttcagccta tgtgtgtctt tataagtgaa 51960
gtgtgtttct tttaggcaac agattaatag gtcttgtttt tccatccagg tcagtaacag 52020
gtcagtatgt cttttgattg gagattttat tccatttaca ttcagtgtta ttattgataa 52080
gtaaggactt acccatgccc ctttgttatt tgttttctgg ttgttttgtg gacttctctt 52140
ccttctttca tttcttcctg tcttccttta ttgaagagaa ttttctccac ttatatgtgt 52200
acagattttt cttaatatct ggtttatggc agttacacat ttgtgcatct gtaaccatcc 52260
tctctttaag tttgcatata cttccagcac tataatttaa atttataatg atgtttggat 52320
accttcatga ttcatatacc cctgaattgc tacaacaaat gtgccatttt tctccttttc 52380
catcagtttt tacttgtgtc ttatcagcta aagtccagga agagattgaa cgtgtgattg 52440
gcagaaaccg gagcccctgc atgcaagaca ggagccacat gccctacaca gatgctgtgg 52500
tgcacgaggt ccagagatac attgaccttc tccccaccag cctgccccat gcagtgacct 52560
gtgacattaa attcagaaac tatctcattc ccaaggtaag tttgtttctc ctacactgca 52620
actccatgtt ttcgaagtcc ccaaattcat agtatcattt ttaaacctct accatcaccg 52680
ggtgagagaa gtgcataact catatgtatg gcagtttaac tggactttct cttgtttcca 52740
gtttggggct ataaaggttt gtaacaggtc ctagtgtctg gcagtgtgtg ttctccagat 52800
ttattatctt tcttcaagat tggtttggct actcttaggt gcttatattt ccaaataatt 52860
tttaaaggta ttagtttgtc aatttcccaa aaccttgggc tggaatttct ggcagggtga 52920
cactaaattt ataggctagt ttggaaagaa ctgaatcttg acacgttgag gctttccatt 52980
cctgaatata attatgcttc caatttgttt ggggtttctt ttatttaacc aggaatgttg 53040
tgaatttgtt gtcatggctt tcgagtcttt ggttttccct agataattaa tatttttgtt 53100
gtagaacata aatagttttt atcattctga tgatgttaat ctgtcaactt tgctaaattt 53160

actagtcact attcgtaatt tatttctgga ttcattgtaa tttctgtgta tattatactg 53220
tatctgagtt aatattgttt tatttcttat tttccatttc tcatgggctt aatgtctctt 53280
tatcgcattc attattgcat tagctagaat ttctaggaga gcattgaata gaattggtga 53340
cagtggggat ccttgtttct catttctaat ctgcaggaag cagtggaagt tttccatttc 53400
aatattgaga atgatgcttg aagtagattt tggtagatat tttttatcag attagagaag 53460
tttgctgtca tatatatata tgtcataatt gtgtgtaaaa tcttgtcaaa tcaacactct 53520
gcatctattt atataattgt gtgttagtcc atttccattg ctataaagga atatctttga 53580
ctgggtaatt tataaataaa agagtttcat tttggctcat ggctctgcag gctgtgcaag 53640
aggcataatg tgggcaggca tctactccta gtaaggactt caggtagctt tcaatcttcg 53700
gagaaggtga aggggaagca agcatgtcac atggcaagag agggagtaag agaaaaagga 53760
gtagaagtga ctttaaacaa ccagctagtg tgtgaactaa cagaatatga actcacttat 53820
tatcatggga agaggtttaa gccactcatg aagaattcac ccccatgtcc taaatacttc 53880
tcacaccaga acccacctcc aacattgaga atcacatttc aacatgagat ttggagggga 53940
caaatatcca cacaacatca gattatatat attgggctta catttctcac taaatagaaa 54000
taagtatgca catagagatg aactggcctt cttcatattg tttaggactt cagtgtttac 54060
attcacaaga gatttggttt atagttttat ttctcagaat tttcaagatt tttgatatta 54120
agattatgcc agccttataa aactatgcaa gaaatgtttc ctaatgtgag actgttttta 54180
ttgccgttcc ctctccccac ttttcttctt gtgatcactt agttgtatct ttgaaatgcc 54240
tgataacttt tggttaaata ccagatggta tatgtaagaa attgcagaat tgcagaggag 54300
gtttatattc ttcaaagaaa gattcattcc tctcccatcg gcagtcataa tgacatgata 54360
cctgttgtca tgattcagaa ttggacataa tgtgaatcca gttgaaaatt gagtgtcagt 54420
gttagcttag ctctctgagg ttctcctacc tttggaagct tgagaccaat ttttgtcttg 54480
acagctttgc agtgccttct atcacatggt ttctgtattt atccagacac tccatttgcc 54540
atctcaaacc tactcttttc caatcaaaac caatatatat tattagtaaa ataaaaaata 54600
atataactga aagttgtatt ttcaagggga aaaagcaatg agacttggta aggttgcaat 54660
tactaatgtc atcttttgaa gaagacagaa ttgataattt acttagagat tatggggctt 54720
accctagtgg tcactgagtt agtacaagag aaaagacttc aatgcactct gttttcacaa 54780
acacaggaga taaccaggag actttcgctt ctcaaaatta gggtctgagg gtaatggtaa 54840
tgaaacttac ttcttctcct ataactaatt aaatgagaaa tatactgaag aatcaggcac 54900
gagttgccag tctccccagt tcactgcatc agctttggtg acctcctgga ggggaacgct 54960
gcatctgatg tcttcattcc cccctatgca gggcagctag ctcatgctgt gcctgatgtc 55020
ttttgagccc tgagaagcca gagcccaagg ggattaccca ttgaatccct tcttcgtgga 55080
atagcccagt gcagaaaaaa gcagcactgt gtggccaatt ctaccctctt tacaaatcag 55140
atacatgtgt tcttctgcta caggctggat ccacttatct agctggagat gatagtctga 55200
gacaggagtt ggcaaactgt ctctgtaatg ggccagatag taaatatttt agacttttca 55260

ggcaatgtgg tttctgttgc aattcttgac atctgctatt gtagcatgat agcagtcaca 55320
aacaatctgc aaatgaatga ggattgatat gttccaattt ttatagaaac aaatgtaaat 55380
ttcctataaa atgtttgttt caaaaatatt gtttcttgtt tgcatttgtt caatgtaagt 55440
acttttagct cacatactat acaaaaacag gtggcagggt ggacttgtgc agggagtatt 55500
ttttattgat gcctaatata agacatccct gtccagtaga attttctgca aagatgtgcg 55560
attgatttgt gcttttatgt catatttaaa gaatatttgt cagcacaaga ttccaaagac 55620
tggctttcaa ctttcccaga atttttttgt tttagctctt atatctagat caatgacata 55680
tttcaaatta atttttattt cataatatga agtaatagtg gtcatgcttc aatgtctata 55740
aatttctact tattcaagta acatttgttg aaataaatct gctttggtat cttaataaca 55800
ttttaatata gtacttgttt ataaatattc aagggaatat ttttaaaaaa caaaattata 55860
gactatttat atgaaaattt agggggtaga atttttccta acatttttat tataaaattt 55920
tttaaaataa ataacattac acacacatat gtatatgtac atatggcagt gtgtatacat 55980
atctactctg atatctgtcc atccatctat ttacacctca atacttcagt atgtatagca 56040
caataacaat ggttttctcc tacataataa tagcattcat actgtggagt tattaataca 56100
ataatattat ttactataca gcttttattt aaaatttccc aactttaaaa taatatttta 56160
atcacttttt tcttgcaatt cactctccag tcaagaaata agcacatagt ttcagtatcc 56220
tgttactttc catctcctgt aatcaagaag agtccatttc ctctgtttcc tttcagtatg 56280
ttgatctctg tgaagagttg agcaattagt catttaaatt ttcctggggc tcgttttggg 56340
taattgtttt ctaaatatta tattccagtt atgactttgg caagaaaact acatagtatt 56400
ttgaatattc tgctgaatca cagtggtgag gcaggggcac ataatgtcac atatgccatt 56460
attggttatg ctaagatcct cacctaataa gttggaatct gccagatctt tccattgaag 56520
gggttctgct ttttatgcgt gaatgatgaa aatctatggc atggtacttc aaggcagaac 56580
accctgtttc ccaagtcaca catccaatag ttttatcttt cattaatgat cctcatctga 56640
cctgatgatc ttgacctgaa tggttacaaa taggtgattt ttctaatttc atcattcctt 56700
ccgtattttt aagctaggat taatctgtaa tgaatatctc tcccatatct ttaaatgaag 56760
acaaacaaac tgcatggcaa tgacaagaac tcaatagttt tatccttgcc actacacttg 56820
ttaccatccc agaagatgtc atttgaaatt caatcatatg tttattcaca aatgccctgt 56880
gaacagatac tgtggccatt tcccatgtca acagagactt caacttttag ttatactcta 56940
cacataaggt tggtgaaaac tgaagtgatg gaatagagaa gcaagtgtgg acagccacta 57000
gtttccaagc ttgatgaaaa ggaggacaga ttcagaaggt tgcatccaag tatccaagta 57060
ctcaggactt caaatgtgat tgcagggcac tttagcaaga ttattgtcat gggccttaag 57120
ttcatgactc ttattacttg gtctatccat ctggaaatgg tactgccctt ctttggaacg 57180
ggatttcctc atctgcacat caaaagattt aactacatga ttaccactgt ttcttcaacc 57240
ttcatggctt ctttacagct cagttcatct atgtctcttg tttctagggc acaaccatat 57300

taatttccct gacttctgtg ctacatgaca acaaagaatt tcccaaccca gagatgtttg 57360
accctcatca ctttctggat gaaggtggca attttaagaa aagtaaatac ttcatgcctt 57420
tctcagcagg taatataaat ttatttccat ttgtgtttca gggtacaaga taactttttt 57480
gatccattgg aacttacatg tgcctcctct gcagtggtac aattactctt tgtacatgat 57540
caagagcact gttctgaatg cctgtgtaca ccctgctcat gatacatcct aattattggg 57600
ccagattagt ggactttggg gagttaatcc aattcttcca aattgagaaa gctgaaatat 57660
aggttggttg aattctgcct ctaggtacac cagtgaggta cccaagaact cctcctggaa 57720
gataaaacta attacatttt cctcactagc catgaggaag ttatctcact ccagaacttc 57780
actgagtgtc ttccacatgg tgtccctcac cccctaggct gggcttgtag gataaaatta 57840
tccacaaaca cagaataggg tcttaaaagg ctcacttctg agtttggaaa gcagagtaaa 57900
cagatcattg tagttcaata ggactgaggc tgtgatatgg aaagaacagg ctgttggggg 57960
ttgggaggta gacggaaaag ctgtctgctt cttgtactct tataccccaa agtgaggcat 58020
aagtaatatt ttaatagcag taaagacatt tgagctacct caaaggaggc agagaggatg 58080
aaaagaagag aagacagggc tattaaagga gataatgagc cacaggagca ggacattggc 58140
tctaaataaa ggatattaga acctttgcca aatgaccatg gatgggatga gggggacatt 58200
gggaatgtag caggatactc tgcagtgatg cagagcacca tgctgttccc ctagtcatgg 58260
ccatgtatat tggatgttgg atctccatac ttgaaatgtg tgtgctgagc atctggtgat 58320
agaatctcct tcctttattc tatgaccttt aatgtctgct ttatatctgc cactgtagat 58380
accaagacaa caagaaaaga aaccttcctt caagcattca catttagcac atgtattagt 58440
ccactcttac attgccttaa agaactacct gagattgggt aatttatgaa gaaaagaggt 58500
ttaattcact cacagatcca caggctgtac aggaagcatg gctgggagac ctcaagaaac 58560
ttgcaatcat ggtgggaggc agagcggaag caagcacatc tttccatggc agagcagaag 58620
agagagaaca atgaggaaag gctacatacc tttaaacaac cagatctcat gagaactcac 58680
tcactatcat gagaacagaa agggggaaat tatcccccat gattcaatca cctctcacca 58740
ggtatctccc ccaacattgg gaattacagt ttgacatgag atttgagtaa ggacacagag 58800
cccaaccata tcgcacacta ataaataaat aaataaatca gtaattatta ttctaggtga 58860
tccatgctgt ctgtttcttc tgccataaca aaatatccaa gactaggcaa ttgataaata 58920
atggaaattt atttctcaca gttctgaagg ctgggagttc caagatcgat gtgtcagcaa 58980
gttcagtgtc tgatgtgggc ctgttcctta cagataatgc cccctctgta tccttatatg 59040
gcagaagggc aaaaaggcaa aaggggagaa atggctccct tgcatctctt gatgttatta 59100
ttttagcaag gacagagccc tcctgactta ttcacttcct aaaaggagcc atctctttag 59160
taatgttgca ttgggattat gtgtcaacat atgaaattgg gggaggcata ttcagaccat 59220
agcacatttt tcaatggaaa tataatgttg aggaaaccca gagaaggcaa cattttcttg 59280
ctcaaggaga tgagaaagag ggtaaaaaag gagataaaat ttgacctatg tcctgactgt 59340
ggtaatagaa aagttcatct tggctaaaag gagcagcatg atataaaatt tgaaacctca 59400

tggtgtgttg gagactgatg atgagtggct atgcctagag ttgacagtat cggatttgaa 59460
gagtgtaagg agtgatgtgg atcatcagac tggaaacaga atgtgagggt ccagatcaat 59520
ccattgggac cttatcctat aggacataca gggaagccat ttaaagtttt aaagtgagaa 59580
ggtgacatgt ttagacatgt gctcctgaaa gtacctagag gaaaaaaatc tttggctgca 59640
tattgagcca gaaatacaaa gggaaataca gtatgttagc ctcctcctct aagcccttct 59700
cagttcaacc cactggacaa gaaatgtatg tttctaaaga aagattgatg aagacattta 59760
aagtctcttg aaagatttta ataaagtgct tggcatgtag ctggtactca acaaatattt 59820
gttgaataca gggtgcctgt taagatctga tattaggtga agagtaagta tgtccattca 59880
tttttcagtt gcctatacat ccatccattc atccatttat ccatccactc atccatccat 59940
tcattcatgc atgcacccat ccacccatct atctcttcat ctcttctacg attcactgaa 60000
cagttattgc atattctgtt tgtgccagtt acagagacag tgtttgtcac tgtcacagtt 60060
acgcatgagg agtaactgct ctctgtgttt gctattttca ggaaaacgga tttgtgtggg 60120
agaagccctg gccggcatgg agctgttttt attcctgacc tccattttac agaactttaa 60180
cctgaaatct ctggttgacc caaagaacct tgacaccact ccagttgtca atggatttgc 60240
ctctgtgccg cccttctacc agctgtgctt cattcctgtc tgaagaagag cagatggcct 60300
ggctgctgct gtgcagtccc tgcagctctc tttcctctgg ggcattatcc atctttcact 60360
atctgtaatg ccttttctca cctgtcatct cacattttcc cttccctgaa gatctagtga 60420
acattcgacc tccattacgg agagtttcct atgtttcact gtgcaaatat atctgctatt 60480
ctccatactc tgtaacagtt gcattgactg tcacataatg ctcatactta tctaatgttg 60540
agttattaat atgttattat taaatagaga aatatgattt gtgtattata attcaaaggc 60600
atttcttttc tgcatgttct aaataaaaag cattattatt tgctgagtca gtttattaga 60660
ccttccttct tttatgcata atgtaggtca gaaattaaag aaaatagagt tccaggaggc 60720
cat 60723

<210> 2
<211> 18
<212> DNA
<213> Artificial

<220>
<223> forward primer

<400> 2
cggagcccct gcatgcaa 18

<210> 3
<211> 17
<212> DNA
<213> Artificial

<220>
<223> forward primer

<400> 3

ggagcccctg catgcaa                                                    17


<210>  4
<211>  16
<212>  DNA
<213>  Artificial

<220>
<223>  forward primer

<400>  4
gagcccctgc atgcaa                                                     16


<210>  5
<211>  15
<212>  DNA
<213>  Artificial

<220>
<223>  forward primer

<400>  5
agcccctgca tgcaa                                                      15


<210>  6
<211>  14
<212>  DNA
<213>  Artificial

<220>
<223>  forward primer

<400>  6
gcccctgcat gcaa                                                       14


<210>  7
<211>  36
<212>  DNA
<213>  Artificial

<220>
<223>  reverse primer

<400>  7
gtttaaaaat gatactatga atttggggac ttcgaa                               36


<210>  8
<211>  35
<212>  DNA
<213>  Artificial

<220>
<223>  reverse primer

<400>  8
tttaaaaatg atactatgaa tttggggact tcgaa                                35


<210>  9
<211>  34
<212>  DNA
<213>  Artificial

<220>

<223>  reverse primer

<400>  9
ttaaaaatga tactatgaat ttggggactt cgaa                                    34


<210>  10
<211>  33
<212>  DNA
<213>  Artificial

<220>
<223>  reverse primer

<400>  10
taaaaatgat actatgaatt tggggacttc gaa                                     33


<210>  11
<211>  32
<212>  DNA
<213>  Artificial

<220>
<223>  reverse primer

<400>  11
aaaaatgata ctatgaattt ggggacttcg aa                                      32


<210>  12
<211>  31
<212>  DNA
<213>  Artificial

<220>
<223>  reverse primer

<400>  12
aaaatgatac tatgaatttg gggacttcga a                                       31


<210>  13
<211>  30
<212>  DNA
<213>  Artificial

<220>
<223>  reverse primer

<400>  13
aaatgatact atgaatttgg ggacttcgaa                                         30


<210>  14
<211>  29
<212>  DNA
<213>  Artificial

<220>
<223>  reverse primer

<400>  14
aatgatacta tgaatttggg gacttcgaa                                          29


<210>  15
<211>  28
<212>  DNA

<213>  Artificial

<220>
<223>  reverse primer

<400>  15
atgatactat gaatttgggg acttcgaa                                          28


<210>  16
<211>  27
<212>  DNA
<213>  Artificial

<220>
<223>  reverse primer

<400>  16
tgatactatg aatttgggga cttcgaa                                           27


<210>  17
<211>  26
<212>  DNA
<213>  Artificial

<220>
<223>  reverse primer

<400>  17
gatactatga atttggggac ttcgaa                                            26


<210>  18
<211>  25
<212>  DNA
<213>  Artificial

<220>
<223>  reverse primer

<400>  18
atactatgaa tttggggact tcgaa                                             25


<210>  19
<211>  24
<212>  DNA
<213>  Artificial

<220>
<223>  reverse primer

<400>  19
tactatgaat ttggggactt cgaa                                              24


<210>  20
<211>  23
<212>  DNA
<213>  Artificial

<220>
<223>  reverse primer

<400>  20
actatgaatt tggggacttc gaa                                               23

<210>  21
<211>  22
<212>  DNA
<213>  Artificial

<220>
<223>  reverse primer

<400>  21
ctatgaattt ggggacttcg aa                                                22


<210>  22
<211>  21
<212>  DNA
<213>  Artificial

<220>
<223>  reverse primer

<400>  22
tatgaatttg gggacttcga a                                                 21


<210>  23
<211>  20
<212>  DNA
<213>  Artificial

<220>
<223>  reverse primer

<400>  23
atgaatttgg ggacttcgaa                                                   20


<210>  24
<211>  19
<212>  DNA
<213>  Artificial

<220>
<223>  reverse primer

<400>  24
tgaatttggg gacttcgaa                                                    19


<210>  25
<211>  22
<212>  DNA
<213>  Artificial

<220>
<223>  probe

<400>  25
gtggggagaa ggtcaaggta tc                                                22


<210>  26
<211>  21
<212>  DNA
<213>  Artificial

<220>
<223>  probe

<400>  26

tggggagaag gtcaaggtat c                                                21

<210>  27
<211>  20
<212>  DNA
<213>  Artificial

<220>
<223>  probe

<400>  27
ggggagaagg tcaaggtatc                                                  20

<210>  28
<211>  19
<212>  DNA
<213>  Artificial

<220>
<223>  probe

<400>  28
gggagaaggt caaggtatc                                                   19

<210>  29
<211>  18
<212>  DNA
<213>  Artificial

<220>
<223>  probe

<400>  29
ggagaaggtc aaggtatc                                                    18

<210>  30
<211>  17
<212>  DNA
<213>  Artificial

<220>
<223>  probe

<400>  30
gagaaggtca aggtatc                                                     17

## Description

### Technical Field

The present invention relates to primer sets for amplifying the CYP2C9 gene, reagents for amplifying the CYP2C9 gene containing the same, and the uses thereof.

### Background Art

Cytochrome P450 is an enzyme group that is classified into a super family and includes many subfamilies (for example, CYP1A, CYP1B, CYP2C, CYP2D, CYP2E, CYP3A, etc.). Among them, it is found that a mutation of a gene coding CYP2C9 (the CYP2C9 gene), an isozyme of a human CYP2C subfamily, affects the in vivo dynamics and effect of phenytoin (antiepileptic drug) and warfarin (anticoagulant), which are substrate drugs of CYP2C. CYP2C9*3, a polymorphism of the CYP2C9 gene, is known as a mutation in which isoleucine in position 359 of amino acid (exon 7) has been changed to leucine (Nonpatent Document 1). Position 359 of amino acid, in which this CYP2C9*3 is present, is a site for recognizing substrate drugs, and it affects the functions of an enzyme involved in drug-metabolizing by changing a steric structure (β-strand) thereof due to change of the amino acid. Accordingly, examination of a polymorphism, CYP2C9*3, with respect to the CYP2C9 gene is very important to predict side effects from drugs and to determine the dosing condition that shows excellent effect.

On the other hand, detection of a point mutation, a so-called single nucleotide polymorphism (SNP), is employed widely as a method of analyzing, at the gene level, for example, the causes of all types of diseases and the individual differences in disease liability (susceptibility to diseases) and in drug action. Examples of the common methods of detecting a point mutation include: (1) a direct sequencing method in which the region corresponding to a sequence to be detected in a target DNA of a sample is amplified by a polymerase chain reaction (PCR) and all the gene sequences are analyzed, (2) a RFLP analysis in which the region corresponding to a sequence to be detected in a target DNA of a sample is amplified by PCR, the amplification product thus obtained is cut with a restriction enzyme whose cleaving action differs depending on the presence or absence of the target mutation in the sequence to be detected and then is electrophoresed, and thereby typing is performed, and (3) the ASP-PCR method in which PCR is performed using a primer with a target mutation located at the 3'-end region and the mutation is judged depending on the presence or absence of amplification.

However, since these methods require, for example, purification of DNA extracted from a sample, electrophoresis, and a treatment with a restriction enzyme, they take time and cost. Furthermore, after PCR is performed, it is necessary to open the reaction container once. Accordingly, there is a possibility that the amplification product may contaminate the next reaction system and thereby the analysis accuracy may be deteriorated. Moreover, since it is difficult to automate, a large number of samples cannot be analyzed. Further, the aforementioned ASP-PCR method (3) is less specific, which also is a problem.

Because of these problems, recently, a method of analyzing the melting temperature (Tm) of double-stranded nucleic acid formed of a probe and target nucleic acid is used as a method of detecting a point mutation. Since such a method is performed through, for example, Tm analysis or analysis of the melting curve of the double strand, it is referred to as melting curve analysis. This method is described below. That is, first, a probe complementary to a sequence to be detected containing a target point mutation is used to form a hybrid (double-stranded DNA) between the aforementioned probe and a target single-stranded DNA contained in a detection sample. This hybridization product is heat-treated, and dissociation (melting) of the hybrid accompanying the temperature rise is detected by a change in a signal such as absorbance. The Tm value then is determined based on the result of the detection and the presence or absence of any point mutation is judged accordingly. The higher the homology of the hybridization product, the higher the Tm value, and the lower the homology, the lower the Tm value. Therefore the Tm value (reference value for assessment) is determined beforehand with respect to the hybridization product between the sequence to be detected containing a point mutation and a probe complementary thereto, and then the Tm value (measured value) of the hybridization product between the target single-stranded DNA contained in the detection sample and the aforementioned probe is measured. When the measured value is comparable to the reference value, it is considered as matching, that is, it can be judged that a point mutation is present in the target DNA. On the other hand, when the measured value is lower than the reference value, it is considered as mismatching, that is, it can be judged that no point mutation is present in the target DNA. Furthermore, according to this method, it also is possible to automate the gene analysis.

However, such a detection method using Tm analysis also has a problem in that a region including a site to be detected must be able to be amplified specifically and efficiently in PCR. Particularly, many isozymes are present in CYP and the sequences for coding them also are very similar to one another. Accordingly, there is a possibility that genes coding isozymes other than CYP2C9 also are amplified in PCR. Furthermore, when other isozyme-coding genes also have been amplified as described above, it may cause, for example, a decrease in the reliability of the analysis result. Moreover, as described above, since analysis of one sample is accompanied by a considerable amount of time and energy, it is not practical to analyze a large number of samples, which also is a problem.
[Nonpatent Document 1] PMID: 8873220 Pharmacogenetics. 1996 Aug; 6(4): 341-9.

### Disclosure of Invention

Hence, the present invention is intended to provide primer sets for specifically and efficiently amplifying a target region in the CYP2C9 gene by a gene amplification method.

In order to achieve the aforementioned object, a primer set of the present invention is a primer set for amplifying the CYP2C9 gene by a gene amplification method, wherein the primer set includes the following primer set (1):

### Primer set (1):

a primer set of a pair of primers including a forward primer composed of the following oligonucleotide (F1) and a reverse primer composed of the following oligonucleotide (R1):
(F1): at least one oligonucleotide having a sequence identical to that of a region extending from adenine (A) at base 52466 to be considered as the first base to any one of the 14^{th} to 18^{th} bases in the direction toward the 5' end in the base sequence of SEQ ID NO: 1, with the adenine (A) being the 3' end,
(R1): at least one oligonucleotide complementary to a region extending from thymine (T) at base 52631 to be considered as the first base to any one of the 19^{th} to 36^{th} bases in the direction toward the 3' end in the base sequence of SEQ ID NO: 1, with adenine (A) complementary to the thymine (T) at base 52631 being the 3' end.

A reagent for amplifying a gene of the present invention is a reagent for amplifying the CYP2C9 gene by a gene amplification method, wherein the reagent includes the primer set for amplifying the CYP2C9 gene of the present invention.

A method of manufacturing an amplification product of the present invention is a method of manufacturing an amplification product of the CYP2C9 gene by a gene amplification method, wherein the method includes the following step (I):
(I) amplifying the CYP2C9 gene in a reaction solution using a primer set for amplifying the CYP2C9 gene according to the present invention, with nucleic acid contained in a sample being used as a template.
   A polymorphism analysis method of the present invention is a method of analyzing a polymorphism of a site to be detected in the CYP2C9 gene, wherein the method includes the following steps (i) to (iv):
   (i) amplifying a region including a site to be detected in the CYP2C9 gene in a reaction solution by a method of manufacturing an amplification product of the present invention,
   (ii) preparing a reaction solution that contains the amplification product obtained in step (i) and a probe capable of hybridizing to the site to be detected,
   (iii) measuring signal values that indicate melting states of a hybridization product between the amplification product and the probe while changing the temperature of the reaction solution, and
   (iv) determining a polymorphism of the site to be detected from a change in the signal values accompanying a change in the temperature.

The primer set of the present invention makes it possible to amplify a target region in a reaction solution specifically and efficiently, with the target region including the site where a polymorphism to be detected, CYP2C9*3, is generated in the CYP2C9 gene. Accordingly, the time and cost can be reduced, which is different from the conventional methods described above. Furthermore, as described above, since a region including a site to be detected where CYP2C9*3 is generated can be amplified specifically, for example, further the use of a probe complementary to a sequence to be detected including the site to be detected makes it possible to perform Tm analysis by directly using the aforementioned reaction solution to type the polymorphism. Moreover, since amplification of the target region and typing of the polymorphism can be performed with one reaction solution, it is also possible to automate the operation. Since the use of the primer set of the present invention allows a pretreatment to be omitted even in the case of, for example, a contaminated sample (for instance, whole blood or oral mucosa), the amplification reaction can be carried out quicker and more simply. Furthermore, since the use of the primer set of the present invention allows the amplification reaction to be carried out with higher amplification efficiency as compared to the conventional case, the amplification reaction time also can be shortened. Thus, according to the primer set of the present invention and a reagent including the same as well as the method of manufacturing an amplification product and a polymorphism analysis method, in each of which the primer set and the reagent are used, polymorphism in the CYP2C9 gene can be analyzed quickly and simply, and it therefore can be said that they are very effective in the field of medicine.

### Brief Description of Drawings

FIG. 1 shows graphs indicating the results of Tm analysis in Example 1 of the present invention.
FIG. 2 shows graphs indicating the results of Tm analysis in Example 1 of the present invention described above.
FIG. 3 shows graphs indicating the results of Tm analysis in Example 2 of the present invention.

### Best Mode for Currying Out the Invention

### < Primer set for amplifying CYP2C9 gene>

As described above, the primer set for amplifying the CYP2C9 gene of the present invention is characterized by including the aforementioned primer set (1). This primer set (1) makes it possible, as described above, to amplify specifically and efficiently a target region including a site to be detected where CYP2C9*3 is generated. Therefore, when this region is amplified using the primer set of the present invention, polymorphism in the CYP2C9 gene can be analyzed more efficiently as compared to the conventional cases. Hereinafter, a forward primer also may be referred to as "F primer" and a reverse primer as "R primer".

As described above, the primer set (1) is a primer set of a pair of primers including a forward primer composed of the following oligonucleotide (F1) and a reverse primer composed of the following oligonucleotide (R1):
(F1): at least one oligonucleotide having a sequence identical to that of a region extending from adenine (A) at base 52466 to be considered as the first base to any one of the 14^{th} to 18^{th} bases in the direction toward the 5' end in the base sequence of SEQ ID NO: 1, with the adenine (A) being the 3' end, and
(R1): at least one oligonucleotide complementary to a region extending from thymine (T) at base 52631 to be considered as the first base to any one of the 19^{th} to 36^{th} bases in the direction toward the 3' end in the base sequence of SEQ ID NO: 1, with adenine (A) complementary to the thymine (T) at base 52631 being the 3' end.

The base sequence indicated in SEQ ID NO: 1 is a full-length sequence of human cytochrome P450 family 2 subfamily C polypeptide 9 (CYP2C9) and, for example, has been registered at NCBI under the accession No. AY702706.

The primer set (1) is a primer set for amplifying a DNA strand including a region from base 52467 to base 52630 in SEQ ID NO: 1, as well as a strand complementary thereto. Base 52521 in this region (i.e. base 52521 in SEQ ID NO: 1) is known for the presence of a point mutation (52521A, 52521C) that affects the function of CYP2C9, and the polymorphism thereof is CYP2C9*3 described above. When the CYP2C9 gene is translated to protein, a polymorphism, in which position 359 of amino acid being isoleucine (IIe), is indicated in the case where base 52521 is A, and a polymorphism, in which position 359 of amino acid being leucine (Leu), is indicated in the case where base 52521 is C. In the present invention, the polymorphism of this site can be indicated as 52521A/A or 52521C/C in the case of homozygote and as 52521A/C in the case of heterozygote. Hereinafter, this primer set (1) also may be referred to as a "primer set for CYP2C9*3".

In the present invention, the F1 primer and R1 primer of the primer set (1) can be any primers, as long as the base located at the 3' end that serves to determine the site from which DNA polymerase starts amplification satisfies the aforementioned condition. Fixation of the base located at the 3' end of the aforementioned primers in this manner makes it possible sufficiently to prevent the primer set (1) from being bound to, for example, another similar isozyme gene (for example, CYP2C8 gene, CYP2C17 gene , CYP2C18 gene, or CYP2C19 gene).

As described above, since the F1 primer and R1 primer each can be any primer as long as the base located at the 3' end is fixed, the length itself of each aforementioned primer is not particularly limited and can be adjusted suitably to a common length. The length of the primers is, for example, in the range of 13- to 50-mers, preferably 14- to 45-mers, and more preferably 15- to 40-mers. Specifically, it is preferable that the F1 primer be at least one oligonucleotide having a sequence identical to that of a region extending from adenine (A) at base 52466 to be considered as the first base to any one of the 14^{th} to 18^{th} bases (preferably the 14^{th} to 17^{th} bases and more preferably the 15^{th} to 17^{th} bases) in the direction toward the 5' end in the base sequence of SEQ ID NO: 1. Furthermore, it is preferable that the R1 primer be at least one oligonucleotide complementary to a region extending from thymine (T) at base 52631 to be considered as the first base to any one of the 19^{th} to 36^{th} bases (preferably the 22^{nd} to 30^{th} bases and more preferably the 23^{rd} to 29^{th} bases) in the direction toward the 3' end in the base sequence of SEQ ID NO: 1. Since each 3' end of the F1 primer and the R1 primer is fixed, the region to be elongated from the primer is, for example, a region from base 52467 to base 52630 in SEQ ID NO: 1 as described above. However, the length of the whole amplification product obtained varies according to the length of the primer to be used.

Furthermore, it is not necessary for the R1 primer and the F1 primer to be oligonucleotides perfectly complementary to the base sequence indicated in SEQ ID NO: 1 and to the strand complementary to the base sequence, respectively. In other words, the part excluding the base located at the 3' end in the aforementioned each primer may be different in one to five bases from that of a perfectly complementary oligonucleotide.

Specific examples of the F1 primer and the R1 primer are indicated below but the present invention is not limited thereto. The combination of these F1 primer and R1 primer is not limited by any means. Specifically, however, a primer set (1') is particularly preferable, which includes a F1' primer composed of oligonucleotide of SEQ ID NO: 2 or SEQ ID NO: 4, and a R1' primer composed of oligonucleotide of SEQ ID NO: 14 or SEQ ID NO: 17. "Tm (°C)" indicated below in the table is Tm (°C) obtained when each sequence indicated below in the table was hybridized with the sequence perfectly complementary thereto. The "Tm (°C)" is a value calculated by using MELTCALC software (http://www.meltcalc.com/), with parameters including an oligonucleotide concentration of 0.2 µM and a sodium equivalent (Na eq.) of 50 mM. The Tm value can be calculated by using, for example, conventionally known MELTCALC software (http://www.meltcalc.com/) or also can be determined by the nearest neighbor method (the same applies below).

**[Table 1]**

| Primer | Sequence | Tm(°C) | SEQ ID NO. |
|---|---|---|---|
| F Primer for CYP2C9*3 | 5'-cggagcccctgcatgcaa-3' | 59.4 | 2 |
| | 5'-ggagcccctgcatgcaa-3' | 56.5 | 3 |
| | 5'-gagcccctgcatgcaa-3' | 53.6 | 4 |
| | 5'-agcccctgcatgcaa-3' | 52.1 | 5 |
| | 5'-gcccctgcatgcaa-3' | 50.2 | 6 |
| R Primer for CYP2C9*3 | 5'-gtttaaaaatgatactatgaatttggggacttcgaa-3' | 58.1 | 7 |
| | 5'-tttaaaaatgatactatgaatttggggacttcgaa-3' | 57.5 | 8 |
| | 5'-ttaaaaatgatactatgaatttggggacttcgaa-3' | 57.2 | 9 |
| | 5'-taaaaatgatactatgaatttggggacttcgaa-3' | 56.9 | 10 |
| | 5'-aaaaatgatactatgaatttggggacttcgaa-3' | 57.2 | 11 |
| | 5'-aaaatgatactatgaatttggggacttcgaa-3' | 56.9 | 12 |
| | 5'-aaatgatactatgaatttggggacttcgaa-3' | 56.6 | 13 |
| | 5'-aatgatactatgaatttggggacttcgaa-3' | 56.3 | 14 |
| | 5'-atgatactatgaatttggggacttcgaa-3' | 55.9 | 15 |
| | 5'-tgatactatgaatttggggacttcgaa-3' | 55.7 | 16 |
| | 5'-gatactatgaatttggggacttcgaa-3' | 54.5 | 17 |
| | 5'-atactatgaatttggggacttcgaa-3' | 53.6 | 18 |
| | 5'-tactatgaatttggggacttcgaa-3' | 53.3 | 19 |
| | 5'-actatgaatttggggacttcgaa-3' | 53.5 | 20 |
| | 5'-ctatgaatttggggacttcgaa-3' | 52 | 21 |
| | 5'-tatgaatttggggacttcgaa-3' | 50.9 | 22 |
| | 5'-atgaatttggggacttcgaa-3' | 51 | 23 |
| | 5'-tgaatttggggacttcgaa-3' | 50.5 | 24 |

Furthermore, each primer of the aforementioned primer set (1) may be, for example, one with the 5' end to which any conventionally known sequence has been added in order to increase the amplification reaction temperature.

Preferably, a primer set for amplifying the CYP2C9 gene of the present invention including such a primer set (1) is used, for example, in amplifying the CYP2C9 gene in a biological sample such as a whole blood sample. Particularly, when the primer set for amplifying the CYP2C9 gene of the present invention is used in combination with a probe for detecting a polymorphism as described later, it is preferable that the ratio of the whole blood sample to be added to the reaction solution for amplifying a gene be 0.1 to 0.5 vol%. This will be described later.

### < Reagent for amplifying CYP2C9 gene>

As described above, a reagent for amplifying the CYP2C9 gene of the present invention is a reagent for amplifying the CYP2C9 gene by a gene amplification method, wherein the reagent includes a primer set for amplifying the CYP2C9 gene of the present invention. The reagent for amplifying the CYP2C9 gene of the present invention is characterized by including a primer set of the present invention and, for example, components other than this are not limited by any means.

For example, in order to detect an amplification product obtained by a gene amplification method in which a primer set of the present invention is used, the reagent for amplifying the CYP2C9 gene of the present invention further may include a probe. As described above, the primer set of the present invention allows amplification products of a target region including a site to be detected where CYP2C9*3 is generated to be obtained by a gene amplification method. Accordingly, when a probe complementary to the sequence to be detected in each target region described above is allowed to coexist, for example, the presence or absence of amplification or the genotype (polymorphism) of the site to be detected can be detected by the method described later. Such probes and the method of using them are explained later in the description of the polymorphism analysis method. Furthermore, it is preferable that the reagent for amplifying the CYP2C9 gene of the present invention be used in amplifying the CYP2C9 gene in a biological sample such as whole blood. Particularly, when the reagent for amplifying the CYP2C9 gene of the present invention is used in combination with the probe described above, it is preferable that the ratio of the whole blood sample to be added to the reaction solution for amplifying a gene be 0.1 to 0.5 vol%. In the present invention, the term "sequence to be detected" denotes a sequence including a site (site to be detected) at which a polymorphism is generated.

The form of the reagent for amplifying the CYP2C9 gene of the present invention is not particularly limited and it may be, for example, a liquid reagent containing a primer set for amplifying the CYP2C9 gene of the present invention or a dry reagent that is to be suspended in a solvent before use. Furthermore, the content of the primer set for amplifying the CYP2C9 gene also is not particularly limited.

### <Method of manufacturing amplification product>

As described above, the method of manufacturing an amplification product of the present invention is a method of manufacturing an amplification product of the CYP2C9 gene by a gene amplification method, wherein the method includes the following step (I):
(I) amplifying the CYP2C9 gene in a reaction solution using a primer set for amplifying the CYP2C9 gene of the present invention, with nucleic acid contained in a sample being used as a template.

When a primer set of the present invention is used to perform an amplification reaction in this manner, the target region including the site to be detected of a polymorphism, CYP2C9*3, in the CYP2C9 gene can be amplified specifically and efficiently as described above. The method of manufacturing an amplification product of the present invention is **characterized in that** a primer set of the present invention is used, and, for example, the type of and conditions for the gene amplification method are not limited by any means.

The gene amplification method is not particularly limited as described above. Examples thereof include the polymerase chain reaction (PCR) method, a nucleic acid sequence based amplification (NASBA) method, a transcription-mediated amplification (TMA) method, and a strand displacement amplification (SDA) method. Particularly, the PCR method is preferable. The present invention is described below using the PCR method as an example but is not limited thereby.

The sample to which the present invention is to be applied is not particularly limited as long as it contains, for example, a nucleic acid sequence to serve as a template. However, it is preferable that the present invention be applied to, for example, a contaminated sample. Examples of the contaminated sample include whole blood, cells in the mouth (for example, oral mucosa), somatic cells of nails and hairs, germ cells, expectoration, amniotic fluid, paraffin-embedded tissue, urine, gastric juice (for example, gastric lavage fluid), and suspensions thereof. According to the method of manufacturing an amplification product using a primer set of the present invention, for example, even in the case of a sample (particularly, a biological sample such as whole blood or cells in the mouth) with various contaminants, the method is less subject to the effect thereof and allows the target region in the CYP2C9 gene to be amplified specifically Thus, according to the present invention, even a highly contaminated sample such as whole blood, which is difficult to use in the conventional methods, can be used as it is, for instance, without being pretreated, for example, without being purified. Therefore, it can be said that an amplification product can be prepared quicker as compared to the conventional method also from the viewpoint of the pretreatment of the sample.

The ratio of the sample to be added to the reaction solution is not particularly limited. Specifically, when the sample is a biological sample (for example, a whole blood sample), the lower limit of the ratio thereof to be added to the reaction solution is, for example, preferably at least 0.01 vol%, more preferably at least 0.05 vol%, and further preferably at least 0.1 vol%. Furthermore, the upper limit of the ratio thereof to be added also is not particularly limited and is, for example, preferably 2 vol% or lower, more preferably 1 vol% or lower, and further preferably 0.5 vol% or lower.

When an optical detection to be described later is intended to be performed, particularly, when an optical detection is performed using a labeled probe, it is preferable that the ratio of a biological sample, such as a whole blood sample, to be added be set at, for example, 0.1 to 0.5 vol%. Generally, in the PCR reaction, a heat treatment is carried out to denature DNA (i.e. to dissociate it into a single-stranded DNA). This heat treatment may denature, for example, sugar or protein contained in the sample and thereby may generate an insolubilized precipitate or turbidity. Therefore, when the presence or absence of an amplification product or the genotype (polymorphism) of a site to be detected is to be checked by an optical method, the generation of such a precipitate or turbidity may affect measurement accuracy. However, when the ratio of the whole blood sample to be added to the reaction solution is set in the range described above, for example, generation of, for example, a precipitate due to denaturation can be prevented sufficiently and thereby the accuracy of measurement carried out by the optical method can be improved, although the mechanism thereof is unknown. Furthermore, since it also sufficiently can prevent PCR from being inhibited due to the contaminants contained in a whole blood sample, the amplification efficiency can be improved further. Accordingly, when in addition to the use of a primer set of the present invention, the ratio of the sample such as a whole blood sample to be added is set in the aforementioned range, the need to pretreat the sample also can be omitted.

Furthermore, the ratio of the whole blood sample in the reaction solution can be indicated not in the aforementioned volume ratio (for example, 0.1 to 0.5 vol%) but in a weight ratio of hemoglobin (hereinafter referred to as "Hb"). In this case, the ratio of the whole blood sample in the reaction solution is, for example, preferably in the range of 0.565 to 113 g/L, more preferably in the range of 2.825 to 56.5 g/L, and further preferably in the range of 5.65 to 28.25 g/L, in terms of the amount of Hb. The ratio of the whole blood sample to be added to the reaction solution may satisfy, for example, both the volume ratio and the Hb weight ratio, or one of them.

The whole blood may be any one of, for example, hemolyzed whole blood, unhemolyzed whole blood, anticoagulated whole blood, and whole blood containing coagulated fractions.

In the present invention, the target nucleic acid contained in a sample is, for example, DNA. The aforementioned DNA may be DNA contained originally in the sample, such as a biological sample, or an amplification product DNA obtained through amplification by a gene amplification method. In the latter case, an example thereof is cDNA that is generated from RNA (for example, total RNA or mRNA) contained originally in the sample by a reverse transcription reaction (for instance, reverse transcription PCR (RT-PCR)).

In the method of manufacturing an amplification product of the present invention, it is preferable that albumin further be added to the reaction solution before the start of a gene amplification reaction. Such addition of albumin further can, for example, reduce the effect of generation of a precipitate or turbidity described above and also further can improve the amplification efficiency. Specifically, it is preferable that albumin be added before the amplification reaction in step (I) or a step of dissociation into a single-stranded DNA.

The ratio of albumin to be added to the reaction solution is, for example, in the range of 0.01 to 2 wt%, preferably 0.1 to 1 wt%, and more preferably 0.2 to 0.8 wt%. The albumin is not particularly limited. Examples thereof include bovine serum albumin (BSA), human serum albumin, rat serum albumin, and horse serum albumin. One of them may be used or two or more of them may be used in combination.

Next, a method of manufacturing an amplification product of the present invention is described using an example in which, with respect to a whole blood sample including DNA as target nucleic acid, an amplification product of the aforementioned target region of the CYP2C9 gene is produced by PCR using the primer set of the present invention. The present invention is characterized by using primer sets of the present invention and other configurations and conditions are not limited by any means.

First, a PCR reaction solution is prepared. The ratio of the primer sets of the present invention to be added is not particularly limited. However, it is preferable that F primer of the primer set (1) be added to be 0.1 to 2 µmol/L, more preferably 0.25 to 1.5 µmol/L, and particularly preferably 0.5 to 1 µmol/L. Furthermore, it is preferable that R primer of the primer set (1) be added to be 0.1 to 2 µmol/L, more preferably 0.25 to 1.5 µmol/L, and particularly preferably 0.5 to 1 µmol/L. The ratio (F:R, molar ratio) between the F primer and the R primer to be added to the primer set is not particularly limited. It is, for example, preferably 1 : 0.25 to 1 : 4 and more preferably 1 : 0.5 to 1 : 2.

The ratio of the whole blood sample in the reaction solution is not particularly limited but is preferably in the range described above. The whole blood sample may be added to the reaction solution without being treated or may be added to the reaction solution after being diluted with a solvent such as water or a buffer solution beforehand. When the whole blood sample is diluted beforehand, the dilution ratio is not particularly limited. It can be set so that, for example, the final ratio of the whole blood added to the reaction solution is in the aforementioned range, for example, 1:100 to 1:2000 and preferably 1:200 to 1:1000.

Other composition components in the reaction solution are not particularly limited and can be conventionally known components, whose ratios also are not particularly limited. Examples of the composition components include DNA polymerase, nucleotide (nucleoside triphosphate (dNTP)), and a solvent. Furthermore, as described above, it is preferable that the reaction solution further contain albumin. In the reaction solution, the order of addition of the respective composition components is not limited by any means.

The DNA polymerase is not particularly limited and, for example, a conventionally known thermoduric bacteria-derived polymerase can be used. Specifically, for example, Thermus aquaticus-derived DNA polymerase (USP 4,889,818 and USP 5,079,352) (trade name: Taq polymerase), Thermus thermophilus-derived DNA polymerase (WO 91/09950) (rTth DNA polymerase), Pyrococcus furiosus-derived DNA polymerase (WO 92/9689) (Pfu DNA polymerase; manufactured by Stratagenes), and Thermococcus litoralis-derived DNA polymerase (EP-A 455 430) (Trademark: Vent; manufactured by New England Biolabs) are commercially available. Particularly, Thermus aquaticus-derived thermostable DNA polymerase is preferable.

The ratio of DNA polymerase to be added to the reaction solution is not particularly limited and is, for example, 1 to 100 U/mL, preferably 5 to 50 U/mL, and more preferably 20 to 30 U/mL. With respect to the unit of activity (U) of DNA polymerase, generally, 1 U denotes the activity that allows all 10 nmol of nucleotide to be taken into an acid-insoluble precipitate in 30 minutes at 74°C in a reaction solution for activity measurement, with an activated salmon sperm DNA being used as a template primer. The composition of the reaction solution for activity measurement is, for example, 25 mM TAPS buffer (pH 9.3, 25°C), 50 mM KCl, 2 mM MgCl₂, 1 mM mercaptoethanol, 200 µM dATP, 200 µM dGTP, 200 µM dTTP, 100 µM [α-³²P] dCTP, and 0.25 mg/mL activated salmon sperm DNA.

Generally, examples of the nucleoside triphosphate include dNTP (dATP, dCTP, dTTP). The ratio of dNTP to be added to the reaction solution is not particularly limited and is, for example, 0.01 to 1 mmol/L, preferably 0.05 to 0.5 mmol/L, and more preferably 0.1 to 0.3 mmol/L.

Examples of the solvent include buffer solutions such as Tris-HCl, Tricine, MES, MOPS, HEPES, and CAPS. Commercially available PCR buffer solutions or buffer solutions of commercially available PCR kits can be used.

Furthermore, the PCR reaction solution further may contain heparin, betaine, KCl, MgCl₂, MgSO₄, glycerol, etc. The ratios thereof to be added can be set in ranges in which the PCR reaction is not inhibited.

The total volume of the reaction solution is not particularly limited and can be determined suitably according to, for example, the equipment (thermal cycler) to be used. It is generally 1 to 500 µL and preferably 10 to 100 µL.

Subsequently, PCR is performed. The cycle conditions in PCR are not particularly limited, and, for example, (1) dissociation of whole blood-derived double-stranded DNA into single-stranded DNA, (2) annealing of a primer, and (3) elongation of a primer (polymerase reaction) are as described below. Furthermore, the number of cycles also is not particularly limited but preferably is at least 30, with the following three steps (1) to (3) being considered as one cycle. The upper limit thereof, in total, is not particularly limited and, for example, 100 cycles or less, preferably 70 cycles or less, and further preferably 50 cycles or less. The change in temperature in each step can be controlled automatically using, for example, a thermal cycler. When primer sets of the present invention are used, since they are excellent in amplification efficiency as described above, 50 cycles can be completed in approximately one hour (preferably within one hour) according to the present invention, while it takes approximately three hours to complete 50 cycles according to the conventional methods.

**[Table 2]**

| | Temperature (°C) and Time (sec) |
|---|---|
| (1) Dissociation of single-stranded DNA | For example, 90 to 99°C, 1 to 120 sec |
| | Preferably, 92 to 95°C, 1 to 60 sec |
| (2) Annealing of primer | For example, 40 to 70°C, 1 to 300 sec |
| | Preferably, 50 to 70°C, 5 to 60 sec |
| (3) Elongation reaction | For example, 50 to 80°C, 1 to 300 sec |
| | Preferably, 50 to 75°C, 5 to 60 sec |

In the manner described above, amplification products complementary to the region including the site to be detected of CYP2C9*3 in the CYP2C9 gene can be produced.

The method of manufacturing an amplification product of the present invention further may include a step of detecting an amplification product by the aforementioned amplification reaction. This makes it possible to detect the presence or absence of the amplification product or the genotype (polymorphism, CYP2C9*3) in the target region in the CYP2C9 gene. The presence or absence of the amplification product can be checked by a conventionally known method. Specifically, it can be checked by, for example, further adding a fluorescently-labeled probe having a sequence complementary to a sequence to be detected in the aforementioned target region to the reaction solution in step (I), and further in step (II), measuring the fluorescence intensity of the fluorescent label in the probe with respect to the reaction solution. Detection of a polymorphism, CYP2C9*3, in the CYP2C9 gene is described below as an embodiment of the present invention.

### < CYP2C9 gene polymorphism analysis method>

A CYP2C9 gene polymorphism analysis method of the present invention is a method of analyzing the polymorphism (CYP2C9*3) of a site to be detected in the CYP2C9 gene, wherein the method includes the following steps (i) to (iv):
(i) amplifying a region including a site to be detected in the CYP2C9 gene in a reaction solution by a method of manufacturing an amplification product according to the present invention,
(ii) preparing a reaction solution that contains the amplification product obtained in step (i) and a probe capable of hybridizing to the site to be detected,
(iii) measuring signal values that indicate melting states of a hybridization product between the amplification product and the probe while changing the temperature of the reaction solution, and
(iv) determining a polymorphism of the site to be detected from a change in the signal values accompanying a change in the temperature.

In this manner, when an amplification product is produced using a primer set of the present invention, it is possible to amplify the region including base to be detected of a polymorphism, CYP2C9*3, in the CYP2C9 gene as described above.

The probe to be used in step (ii) is not particularly limited. For example, a probe is complementary to the sequence to be detected including the site where a polymorphism CYP2C9*3 is generated (hereinafter, also referred to as a "probe for CYP2C9*3"). Preferably, this probe is a probe complementary to a sequence to be detected containing the aforementioned site be detected.

The probes for detecting the polymorphism are not particularly limited and can be configured by a conventionally known method. For instance, they each may be designed as a sequence to be detected containing a site to be detected of a polymorphism, based on the sequence of a sense strand or the sequence of an antisense strand of the CYP2C9 gene. Furthermore, the base located at the site to be detected of a polymorphism can be determined suitably according to the type of polymorphism. In other words, in the case of CYP2C9*3, since the polymorphism of "A" and "C" at base 52521 in SEQ ID NO: 1 have been known, examples of the probe include a probe complementary to either a sequence to be detected including A at base 52521 or a sequence to be detected including C at base 52521 (a probe for detecting a sense strand), and a probe complementary to a sequence of an antisense strand thereof (a probe for detecting an antisense strand). As described above, when a probe is designed, with the base located at the site to be detected where a polymorphism is generated being set to be any one of the bases as described above, it is also possible to judge what type of polymorphism is expressed at a site to be detected in a CYP2C9 gene by the method as described later.

The probe can be added to an amplified reaction solution after step (i) i.e. after a target region in the CYP2C9 gene is subjected to an amplification reaction. However, it is preferable that the probe be added to a reaction solution before the amplification reaction in step (i) since this allows analysis to be performed easily and quickly.

The ratio of the probe to be added to the reaction solution is not particularly limited. For example, a probe is added to be preferably in the range of 10 to 400 nmol/L and more preferably in the range of 20 to 200 nmol/L. When a fluorescent dye is used as the label for a probe, an unlabeled probe with a sequence identical to that of the labeled probe may be used in combination with the labeled probe, for example, in order to adjust the fluorescence intensity to be detected, and the unlabeled probe may include a phosphate group added to the 3' end thereof. In this case, the molar ratio between the labeled probe and the unlabeled probe is preferably, for example, 1 : 10 to 10 : 1. The length of the probe is not particularly limited. It is, for example, 5- to 50-mers and preferably 10- to 30-mers.

The Tm value is described. When a solution containing double-stranded DNA is heated, the absorbance at 260 nm increases. This is because heating releases the hydrogen bonds between both strands in the double-stranded DNA to dissociate it into single-stranded DNA (i.e. DNA melting). When all double-stranded DNAs are dissociated into single-stranded DNAs, the absorbance thereof indicates approximately 1.5 times that obtained at the start of heating (i.e. absorbance of only double-stranded DNAs), which makes it possible to judge that melting is completed thereby. Based on this phenomenon, the melting temperature Tm generally is defined as a temperature at which the absorbance has reached 50% of the total increase in absorbance.

In the aforementioned step (iii), the measurement of the signal values that indicate the melting states of the hybridization product between the amplification product and the probe may be a measurement of absorbance at 260 nm as described above but may be a measurement of the signal of a labeling substance. Specifically, it is preferable that a labeled probe labeled with a labeling substance be used as the aforementioned probe to perform the measurement of the signal of the labeling substance. The labeled probe can be, for example, a labeled probe that exhibits a signal independently but does not exhibit a signal after hybridization, or a labeled probe that does not exhibit a signal independently but exhibits a signal after hybridization. The former probe does not exhibit a signal after forming a hybrid (double-stranded DNA) with a sequence to be detected but exhibits a signal when the probe is released by heating. On the other hand, the latter probe exhibits a signal after forming a hybrid (double-stranded DNA) with a sequence to be detected but the signal is reduced (quenched) when the probe is released by heating. Accordingly, when the signal exhibited by the label is detected under a condition (absorption wavelength etc.) specific to the signal, the progress of melting of the hybridization product and the Tm value can be determined as in the case of the measurement of absorbance at 260 nm.

Specific examples of labeling substances in the labeled probes include a fluorescent dye (fluorophore). A specific example of the labeled probes is preferably a probe that, for example, has been labeled with a fluorescent dye (fluorophore), exhibits fluorescence independently, and allows fluorescence to be reduced (for example, quenched) after hybridization. With respect to a probe that utilizes such a fluorescence quenching phenomenon, particularly, a probe, which is designed so that the 3' end or 5' end of oligonucleotide be C and is labeled with a fluorescent dye whose emission decreases when the end base C thereof approaches G, is preferable. Generally, such a probe is referred to as a guanine quenching probe and is known as so-called QProbe (registered trademark). The use of such a probe makes it possible to verify hybridization and dissociation easily according to a change in the signal.

The fluorescent dye is not particularly limited. Examples thereof include fluorescein, phosphor, rhodamine, and polymethine dye derivative. Examples of commercially available fluorescent dye include BODIPY FL (brand name, manufactured by Molecular Probe Inc.), FluorePrime (trade name, manufactured by Amersham Pharmacia), Fluoredite (trade name, manufactured by Millipore Corporation), FAM (manufactured by ABI), Cy3 and Cy5 (manufactured by Amersham Pharmacia), and TAMRA (manufactured by Molecular Probe Inc.). The detection conditions are not particularly limited and can be determined suitably according to fluorescent dyes to be used. For example, Pacific Blue can be detected with a detection wavelength of 450 to 480 nm, TAMRA can be detected with a detection wavelength of 585 to 700nm and BODIPY FL can be detected with a detection wavelength of 515 to 555 nm.

Specific examples of the sequences of a probe for detecting the polymorphism, CYP2C9*3, is indicated below, but the present invention is not limited thereto. The following probe is a probe for detecting a sense strand.

### Probe

At least one oligonucleotide complementary to a region extending from guanine (G) at base 52516 to be considered as the first base to any one of the 17^{th} to 22^{nd} bases in the direction toward the 3' end in SEQ ID NO: 1, with cytosine complementary to the guanine being the 3' end.

In the aforementioned probe, the base complementary to base 52521 in SEQ ID NO: 1 is represented by "k" and the "k" may be either G or T.

Specific examples of the aforementioned probe is indicated in the following table. "Tm(°C)" indicated below in the table is Tm(°C) obtained when each sequence indicated below in the table was hybridized with the sequence perfectly complementary thereto. The "Tm(°C)" is a value calculated by using MELTCALC software (http://www.meltcalc.com/), with parameters including an oligonucleotide concentration of 0.2 µM and a sodium equivalent (Na eq.) of 50 mM.

**[Table 3]**

| Probe | Sequence | Tm(°C) | SEQ ID NO. |
|---|---|---|---|
| Probe for CYP2C9*3 | 5'-gtggggagaaggtcaaGgtatc-3' | 55.7 | 25 |
| | 5'-tggggagaaggtcaaGgtatc-3' | 54.4 | 26 |
| | 5'-ggggagaaggtcaaGgtatc-3' | 52.8 | 27 |
| | 5'-gggagaaggtcaaGgtatc-3' | 50.2 | 28 |
| | 5'-ggagaaggtcaaGgtatc-3' | 47.3 | 29 |
| | 5'-gagaaggtcaaGgtatc-3' | 44.1 | 30 |

Each probe indicated in the above table consists of a sequence complementary to a region having C at base 52521 in SEQ ID NO: 1, and the capitalized base indicates the base complementary to base 52521 in SEQ ID NO: 1. In each probe, the capitalized base can be replaced by "k", and the "k" may be either G or T. As described above, specific examples of the probe according to the present invention may be strands complementary to oligonucleotides indicated in the above table.

The aforementioned probes are examples and the present invention is not limited thereto. Among the aforementioned probes, oligonucleotide consisting of the base sequence of SEQ ID NO: 28 or SEQ ID NO: 29 is preferable for the probe for CYP2C9. For instance, when the probes indicated in the above table are guanine quenching probes, it is preferable that the cytosine at the 3' end thereof be labeled with the aforementioned fluorescent dye (for instance, BODIPY FL, TAMRA, etc.). Furthermore, a probe with the 5' end labeled with a fluorescent dye may have the 3' end, to which a phosphate group further may be added, in order to prevent the probe itself from elongating.

Next, with respect to the detection method of the present invention, a method of detecting a polymorphism, CYP2C9*3, in the CYP2C9 gene using the following probes is described as an example. However, the present invention is not limited thereto.

### <Probe>

### Probe for CYP2C9*3

5'-gggagaaggtcaaggtatc-(BODIPY FL)-3' (SEQ ID NO: 28), or
5'-ggagaaggtcaaGgtatc-(BODIPY FL)-3' (SEQ ID NO: 29)

First, using a reaction solution containing the aforementioned labeled probes added thereto, PCR was performed as described above, and thereby the target region of the CYP2C9 gene is amplified in the reaction solution. The reaction solution contains, for example, a primer set for amplifying the CYP2C9 gene of the present invention, DNA polymerase, dNTP, a sample containing nucleic acid to serve as a template, and the aforementioned probes. In addition to them, various additives that can be used for amplifying nucleic acid may be contained.

Next, the amplification products thus obtained are dissociated and then single-stranded DNA obtained through dissociation is hybridized with the labeled probes. This can be carried out through, for example, a change in the temperature of the reaction solution.

The heating temperature in the dissociation step is not particularly limited as long as it allows the amplification products to be dissociated. It is, for example, 85 to 95°C. The heating time also is not particularly limited and generally is 1 second to 10 minutes and preferably 1 second to 5 minutes.

The dissociated single-stranded DNAs can be hybridized with the labeled probes by, for example, decreasing the heating temperature employed in the dissociation step after the dissociation step. The temperature condition is, for example, 40 to 50°C.

The temperature of the reaction solution is changed and thereby signal values that indicate the melting states of hybridization products between the amplification products and the labeled probes are measured. Specifically, for example, the reaction solution (the hybridization products between the single-stranded DNAs and the labeled probes) is heated, and thereby the change in the signal values accompanying the temperature rise is measured. As described above, when, for example, a probe (guanine quenching probe), in which the base C at the end has been labeled, is used, fluorescence decreases (or quenches) in the state where the probe has been hybridized with the single-stranded DNA, while fluorescence is emitted in the state where the probe has been dissociated. Accordingly, for example, the hybridization product whose fluorescence has decreased (or quenched) is heated gradually and the increase in fluorescence intensity accompanying the temperature rise may be measured.

The temperature range in which the change in fluorescence intensity is to be measured is not particularly limited. For example, the start temperature is room temperature to 85°C and preferably 25 to 70°C, while the end temperature is, for example, 40 to 105°C. Furthermore, the rate of temperature rise is not particularly limited and is, for example, 0.1 to 20°C/sec and preferably 0.3 to 5°C/sec.

Next, the Tm value is determined by analyzing a change in the signal. Specifically, the amount of change in the fluorescence intensity per unit time at each temperature (-d fluorescence intensity increase/dt) is calculated from the fluorescence intensity obtained and the temperature at which the lowest value is obtained is determined as the Tm value. It is also possible to determine, as the Tm value, the point at which the amount of increase in the fluorescence intensity per unit time (fluorescence intensity increase/t) is the highest. On the contrary, the amount of decrease in the fluorescence intensity is measured when the labeled probe used is not a quenching probe but a probe that does not exhibit a signal independently but exhibits a signal after hybridization.

From this Tm value, the genotype in the site to be detected is determined. In the Tm analysis, the case of a perfectly complementary hybrid (match) results in a higher Tm value indicating dissociation than that obtained in the case of a hybrid including a different single base (mismatch). Accordingly, with respect to the probe, when the Tm value obtained in the case of a perfectly complementary hybrid and the Tm value obtained in the case of a hybrid including a different single base are determined beforehand, the genotype at the aforementioned site to be detected can be determined. For example, in the case where the base located at the site to be detected is assumed to be of a mutation type (with, for instance, C at base 52521 in SEQ ID NO: 1), when using a probe complementary to the sequence to be detected containing the base, the polymorphism of the amplification product can be judged as a mutation type if the Tm value of the resultant hybrid is equal to the Tm value of a perfectly complementary hybrid. Furthermore, the polymorphism of the amplification product can be judged as a wildtype (with, for example, A at base 52521 in SEQ ID NO: 1) if the Tm value of the resultant hybrid is equal to the Tm value of the hybrid including a different single base (i.e. a lower value than the Tm value of the perfectly complementary hybrid). Moreover, when both the Tm values are detected, it can be judged as heterozygote. Thus, the genotype of the polymorphism, CYP2C9*3, can be judged from the Tm value with respect to the labeled probes.

In the present invention, for example, a change in the signal during hybridization may be measured instead of the method in which the temperature of a reaction solution containing the probes is increased (a hybridization product is heated) and a change in the signal accompanying the temperature rise is measured as described above. In other words, when the temperature of the reaction solution containing the aforementioned probes is decreased to form hybridization products, the change in the signal accompanying the temperature decrease may be measured.

Specifically, when using a labeled probe that exhibits a signal independently but does not exhibit a signal after hybridization (for example, a guanine quenching probe), the labeled probe emits fluorescence in the state where single-stranded DNA and the probe are dissociated, but the fluorescence decreases (or quenches) when a hybrid is formed through temperature decrease. Accordingly, for example, the temperature of the reaction solution is decreased gradually and the decrease in fluorescence intensity accompanying the temperature decrease may be measured. On the other hand, when using a labeled probe that does not exhibit a signal independently but exhibits a signal after hybridization, the labeled probe does not emit fluorescence in the state where single-stranded DNA and the probe are dissociated, but the fluorescence is emitted when a hybrid is formed through temperature decrease. Accordingly, for example, the temperature of the reaction solution is decreased gradually and thereby the increase in fluorescence intensity accompanying the temperature decrease may be measured.

Next, examples of the present invention are described. However, the present invention is not limited by the following examples.

### [Example 1]

Blood was collected from nine subjects using heparin lithium blood collection tubes (Samples 1 to 9). Subsequently, 10 µL of blood thus obtained and 90 µL of distilled water were mixed together. Further, 10 µL of this mixture and 90 µL of distilled water were mixed together. Thereafter, 10 µL of the mixture was added to 40 µL of PCR reaction solution having the following composition, and then PCR was performed using a thermal cycler. Conditions for PCR were as follows. That is, after treating at 95°C for 60 seconds, one cycle of treatment at 95°C for 1 second and at 52°C for 10 seconds was repeated for 50 cycles, and further it was treated at 95°C for 1 second and at 40°C for 60 seconds. Subsequently, the PCR reaction solution was heated from 40°C to 95°C at a rate of temperature rise of 1°C/3 seconds, and the change in fluorescence intensity over time was measured. The measurement wavelength was 515 to 555 nm (for detection of the fluorescent dye, BODIPY FL). The time required for 50 cycles of PCR was approximately one hour.

**[Table 4]**

| <PCR reaction solution; unit: µl> | |
|---|---|
| Distilled water | 8.75 |
| 5% NaN₃ | 0.5 |
| 20% BSA | 1 |
| 40% Glycerol | 18.75 |
| 10 × Gene Taq buffer * | 5 |
| 2.5 mM dNTPs | 4 |
| 5 µM probe for CYP2C9*3 | 1 |
| 100 µM CYP2C9 F1 primer | 0.5 |
| 100 µM CYP2C9 R1 primer | 0.25 |
| 5 U/µl Gene Taq FP * | 0.25 |
| Total | 40 µL |

| | |
|---|---|
| * Trade name, Gene Taq FP: manufactured by Nippon Gene Co., Ltd. | |

### <Probe>

### Probe for CYP2C9*3

5'-gggagaaggtcaaggtatc-(BODIPY FL)- 3' (SEQ ID NO: 28)

### <Primer set>

### CYP2C9*3 F1 primer

5'-gagcccctgcatgcaa-3' (SEQ ID NO: 4)

### CYP2C9*3 R1 primer

5'-gatactatgaatttggggacttcgaa-3' (SEQ ID NO: 17)

The Tm value of a hybrid that matches with the probe for CYP2C9*3 is 59°C and that of a hybrid that mismatches therewith is 54°C.

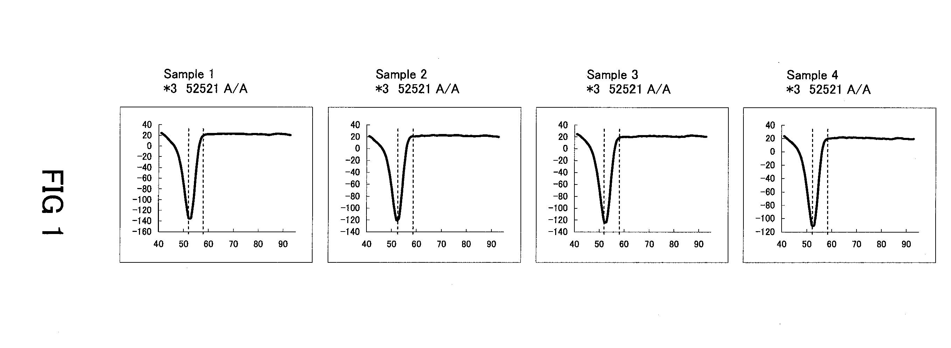

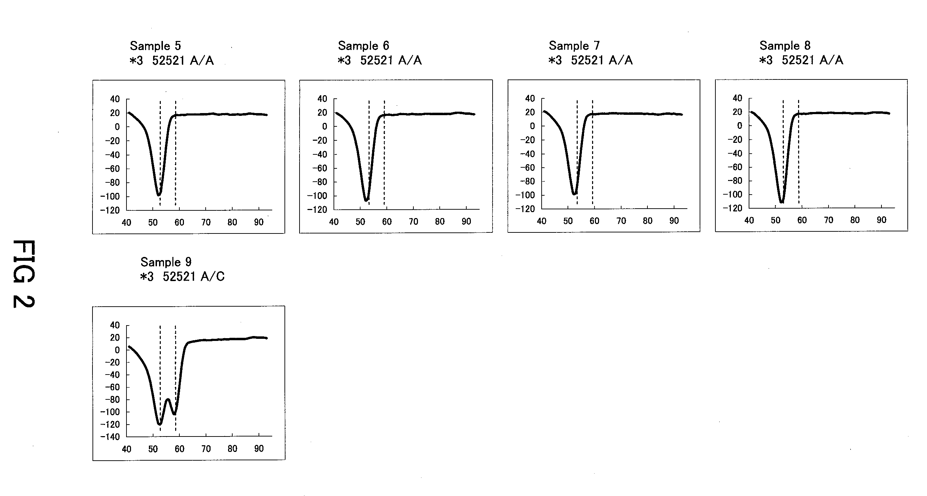

Results of Samples 1 to 9 are indicated in FIGs. 1 and 2. These figures show graphs of Tm analysis that indicate the changes in fluorescence intensity accompanying temperature rise. The differential value of the vertical axis indicates "-d fluorescence intensity increase/dt", while the horizontal axis indicates temperature (the same applies below). As shown in these graphs, the genotype of CYP2C9*3 in each sample was determined from the peaks of the signals. In order to support the results of these examples, with respect to nine subjects, the genotype of CYP2C9*3 was confirmed by the RFLP method. As a result, the same results as those obtained in the example were obtained. Accordingly, the use of a primer set of the present invention made it possible to specifically and efficiently amplify the target region including the site to be detected of CYP2C9*3 in the CYP2C9 gene using a whole blood sample that had not been pretreated and to analyze the polymorphism.

### [Example 2]

Blood was collected from two subjects using EDTA blood collection tubes (Samples 1 and 2). Subsequently, 10 µL of blood thus obtained and 70 µL of diluent A described below were mixed together. Further, 10 µL of this mixture and 70 µL of diluent B described below were mixed together. Subsequently, 10 µL of the mixture thus obtained was heat-treated at 95°C for five minutes. Thereafter, this was added to 46 µL of PCR reaction solution having the following composition, and then PCR was performed using a thermal cycler. The conditions for PCR were as follows. That is, after treating at 95°C for 60 seconds, one cycle of treatment at 95°C for 1 second and at 58°C for 15 seconds was repeated for 50 cycles, and further it was treated at 95°C for 1 second and at 40°C for 60 seconds. Subsequently, the PCR reaction solution was heated from 40°C to 75°C at a rate of temperature rise of 1°C/3 seconds, and the change in fluorescence intensity over time was measured. The measurement wavelength was 515 to 555 nm (for detection of the fluorescent dye, BODIPY FL).

### <Diluent A>

10 mM Tris-HCl (pH 8), 0.1 mM EDTA, 0.05% NaN₃, 0.3% SDS

### <Diluent B>

10 mM Tris-HCl (pH 8), 0.1 mM EDTA, 0.05% NaN₃

**[Table 5]**

| <PCR reaction solution; unit: µl> | |
|---|---|
| Distilled water | 18 |
| 5% NaN₃ | 0.5 |
| 20% BSA | 0.5 |
| 40% Glycerol | 15 |
| 10 × Gene Taq buffer * | 5 |
| 2.5 mM dNTPs | 4 |
| 5 µM probe for CYP2C9*3 | 2 |
| 100 µM CYP2C9 F1 primer | 0.5 |
| 100 µM CYP2C9 R1 primer | 0.25 |
| 5 U/µl Gene Taq FP * | 0.25 |
| Total | 46 µL |

| | |
|---|---|
| * Trade name, Gene Taq FP: manufactured by Nippon Gene Co., Ltd. | |

### <Probe>

### Probe for CYP2C9*3

5'-ggagaaggtcaaggtatc-(BODIPY FL)-3' (SEQ ID NO: 29)

### <Primer set>

### CYP2C9*3 F1 primer

5'-cggagcccctgcatgcaa-3' (SEQ ID NO: 2)

### CYP2C9*3 R1 primer

5'-aatgatactatgaatttggggacttcgaa-3' (SEQ ID NO: 14)

The Tm value of a hybrid that matches with the probe for CYP2C9*3 is 56°C and that of a hybrid that mismatches therewith is 50°C.

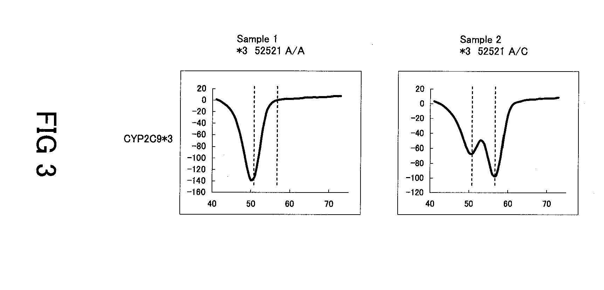

Results of Samples 1 and 2 are indicated in FIG. 3. FIG. 3 shows graphs of Tm analysis that indicate the changes in fluorescence intensity accompanying temperature rise. The differential value of the vertical axis indicates "-d fluorescence intensity increase/dt", while the horizontal axis indicates temperature. As shown in these graphs, the genotype of CYP2C9*3 in the sample was determined from the peaks of the signals. In order to support the results of this example, with respect to two subjects, the genotype of CYP2C9*3 was confirmed by the RFLP method. As a result, the same results as those obtained in the example were obtained. Accordingly, the use of a primer set of the present invention made it possible specifically and efficiently to amplify the target region including the site to be detected of CYP2C9*3 in the CYP2C9 gene using a whole blood sample that had not been pretreated and to analyze the polymorphism.

### Industrial Applicability

As described above, the primer set of the present invention makes it possible to specifically and efficiently amplify a region including a site where a polymorphism, CYP2C9*3, is generated in the CYP2C9 gene. This allows time and cost to be reduced, which is different from the conventional methods as described above. Furthermore, as described above, since the region including a site to be detected of a polymorphism is amplified specifically, for example, the use of a probe complementary to a sequence to be detected including the site to be detected makes it possible to perform Tm analysis directly using the aforementioned reaction solution to type the polymorphism. Moreover, since amplification and typing can be carried out using one reaction solution, the operation can be automated. The use of the primer set of the present invention allows a pretreatment to be omitted even in the case of, for example, a contaminated sample (for instance, whole blood or oral mucosa), and therefore the amplification reaction can be carried out quicker and more easily. Furthermore, when the primer set of the present invention is used, the amplification reaction can be carried out with higher amplification efficiency as compared to conventional cases and thus the reaction time can also be shortened. According to the primer set of the present invention, the reagent including the same, as well as the method of manufacturing an amplification product using them, since the polymorphism in the CYP2C9 gene can be analyzed quickly and simply, it can be said that they are considerably effective in the field of medicine.
## Claims

1. A primer set for amplifying the CYP2C9 gene by a gene amplification method,
wherein the primer set includes the following primer set (1):
Primer set (1):
a primer set of a pair of primers including a forward primer composed of the following oligonucleotide (F1) and a reverse primer composed of the following oligonucleotide (R1):
(F1): at least one oligonucleotide having a sequence identical to that of a region extending from adenine (A) at base 52466 to be considered as the first base to any one of the 14^{th} to 18^{th} bases in the direction toward the 5' end in the base sequence of SEQ ID NO: 1, with the adenine (A) being the 3' end,
(R1): at least one oligonucleotide complementary to a region extending from thymine (T) at base 52631 to be considered as the first base to any one of the 19^{th} to 36^{th} bases in the direction toward the 3' end in the base sequence of SEQ ID NO: 1, with adenine (A) complementary to the thymine (T) at base 52631 being the 3' end.

2. The primer set for amplifying the CYP2C9 gene according to claim 1, wherein the primer set (1) is the following primer set (1'):
Primer set (1'):
a primer set of a pair of primers including a forward primer composed of the following oligonucleotide (F1') and a reverse primer composed of the following oligonucleotide (R1'):
(F1'): at least one oligonucleotide selected from oligonucleotide consisting of the base sequence of SEQ ID NO: 2 and oligonucleotide consisting of the base sequence of SEQ ID NO: 4, and
(R1'): at least one oligonucleotide selected from oligonucleotide consisting of the base sequence of SEQ ID NO: 14 and oligonucleotide consisting of the base sequence of SEQ ID NO: 17.

3. The primer set for amplifying the CYP2C9 gene according to claim 1, wherein the primer set for amplifying the CYP2C9 gene is a primer set for amplifying the CYP2C9 gene in a biological sample.

4. The primer set for amplifying the CYP2C9 gene according to claim 3, wherein the biological sample is whole blood.

5. A reagent for amplifying the CYP2C9 gene by a gene amplification method,
wherein the reagent comprises a primer set for amplifying the CYP2C9 gene according to claim 1.

6. The reagent for amplifying the CYP2C9 gene according to claim 5, further comprising a probe that can hybridize to a site to be detected in the CYP2C9 gene.

7. The reagent for amplifying the CYP2C9 gene according to claim 6, wherein the probe is composed of the following oligonucleotide (P1'):
(P1') at least one oligonucleotide selected from oligonucleotide consisting of the base sequence of SEQ ID NO: 28 and oligonucleotide consisting of the base sequence of SEQ ID NO: 29.

8. The reagent for amplifying the CYP2C9 gene according to claim 6, wherein the probe is a fluorescently-labeled probe.

9. A method of manufacturing an amplification product of the CYP2C9 gene by a gene amplification method,
wherein the method comprises the following process (I):
(I) amplifying the CYP2C9 gene in a reaction solution using a primer set for amplifying the CYP2C9 gene according to claim 1, with nucleic acid contained in a sample being used as a template.

10. The method of manufacturing an amplification product according to claim 9, wherein a probe that can hybridize to a site to be detected in the CYP2C9 gene further is added to the reaction solution in the process (I).

11. The method of manufacturing an amplification product according to claim 10, wherein the probe is composed of the following oligonucleotide (P1'):
(P1') at least one oligonucleotide selected from oligonucleotide consisting of the base sequence of SEQ ID NO: 28 and oligonucleotide consisting of the base sequence of SEQ ID NO: 29.

12. The method of manufacturing an amplification product according to claim 10, wherein the probe is a fluorescently-labeled probe.

13. The method of manufacturing an amplification product according to claim 12, wherein the method further comprises the following process (II):
(II) measuring fluorescence intensity of a fluorescent label contained in the fluorescently-labeled probe in the reaction solution.

14. The method of manufacturing an amplification product according to claim 9, wherein the sample is a biological sample.

15. The method of manufacturing an amplification product according to claim 14, wherein the biological sample is whole blood.

16. The method of manufacturing an amplification product according to claim 15, wherein the ratio of the whole blood sample to be added to the reaction solution is 0.1 to 0.5 vol%.

17. A polymorphism analysis method of analyzing a polymorphism of a site to be detected in the CYP2C9 gene, wherein the method comprises the following processes (i) to (iv):
(i) amplifying a region including a site to be detected in the CYP2C9 gene in a reaction solution by a method of manufacturing an amplification product according to claim 9,
(ii) preparing a reaction solution that contains the amplification product obtained in process (i) and a probe capable of hybridizing to the site to be detected,
(iii) measuring signal values that indicate melting states of a hybridization product between the amplification product and the probe while changing the temperature of the reaction solution, and
(iv) determining a polymorphism of the site to be detected from a change in the signal values accompanying a change in the temperature.

18. The polymorphism analysis method according to claim 17, wherein in the process (i), a probe that can hybridize to the site to be detected is added to the reaction solution prior to an amplification reaction.
